(19) Europäisches Patentamt
European Patent Office
Office européen des brevets

(11) Veröffentlichungsnummer: **0 290 385 B1**

# (12) EUROPÄISCHE PATENTSCHRIFT

(45) Veröffentlichungstag der Patentschrift: **21.10.92**

(51) Int. Cl.5: **C07D 205/08**, C07C 229/22, C07C 227/16, C07D 265/08

(21) Anmeldenummer: **88810264.7**

(22) Anmeldetag: **26.04.88**

## (54) Neues Verfahren zur Herstellung von 4-Acyloxy-3-hydroxyethyl-azetidinonen.

(30) Priorität: **04.05.87 CH 1683/87**

(43) Veröffentlichungstag der Anmeldung:
**09.11.88 Patentblatt 88/45**

(45) Bekanntmachung des Hinweises auf die Patenterteilung:
**21.10.92 Patentblatt 92/43**

(84) Benannte Vertragsstaaten:
**BE CH DE ES FR GB IT LI NL SE**

(56) Entgegenhaltungen:
**EP-A- 0 106 652**
**EP-A- 0 167 154**
**EP-A- 0 171 064**
**EP-A- 0 180 398**
**EP-A- 0 181 831**
**EP-A- 0 210 657**
**EP-A- 0 240 164**
**DE-A- 3 340 006**
**GB-A- 2 144 419**
**US-A- 4 467 107**
**US-A- 4 596 677**

**TETRAHEDRON LETTERS, Band 23, Nr. 22, 1982; REIDER, P.J. et al.: "Total synthesis of thienamycin: A new approach from aspartic acid" Seiten 2293-2296**

**CHEMICAL ABSTRACTS, Band 84, Nr. 25, 21. Juni 1976, Columbus, Ohio, US; RIBALDONE, G. et al.: "Dihydro-1,3-oxazine" Seite 578, Zusammenfassung-Nr. 180 240u**

(73) Patentinhaber: **CIBA-GEIGY AG**
**Klybeckstrasse 141**
**CH-4002 Basel(CH)**

(72) Erfinder: **Schneider, Peter, Dr.**
**Bäumliackerstrasse 8**
**CH-4103 Bottmingen(CH)**
Erfinder: **Ramos, Gerardo, Dr.**
**General Guisan-Strasse 35**
**CH-4144 Arlesheim(CH)**
Erfinder: **Bersier, Jacques, Dr.**
**Gstaltenrainweg 61**
**CH-4125 Riehen(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein neues Verfahren zur Herstellung von 4-Acyloxy-3-hydroxyethyl-azetidinonen, die als Ausgangsstoffe zur Herstellung von β-Lactam-Antibiotika verwendet werden können. Neue Zwischenprodukte sind ebenfalls Gegenstand der Erfindung.

Ein nach dem erfindungsgemässen Verfahren herstellbares (3R,1′R)-4-Acyloxy-3-(1′-hydroxyethyl)-2-azetidinon der Formel I, insbesondere das (3R,4R,1′R)-4-Acetoxy-3-(1′-hydroxyethyl)-2-azetidinon der Formel Ia,

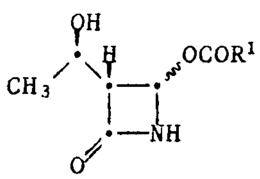

(I)

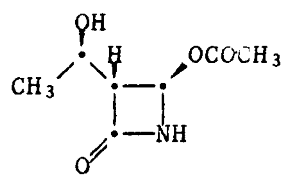

(Ia)

ist als Ausgangsmaterial für die Herstellung einer Vielzahl von hochwirksamen β-Lactam-Antibiotika, beispielsweise von Penemen, Carbapenemen oder entsprechenden Oxapenem-, Penicillin- oder Cephalosporin-Derivaten geeignet. Bei diesen Umsetzungen wird die Acyloxygruppe in 4-Stellung des Azetidinons durch geeignete Schwefel- oder Kohlenstoff-Nukleophile ausgetauscht. Solche Reaktionen sind im Uebersichtsartikel von W. Dürckheimer et. al., Angew. Chem. 97, 183 (1985), erläutert.

Zu Penemen gelangt man beispielsweise, indem man eine Verbindung der Formel I, gegebenenfalls nach Einführung einer Schutzgruppe an der Hydroxygruppe und/oder dem Lactam-Stickstoffatom, mit Mercaptanen, Thiosäuren, Dithiosäuren, Trithiocarbonaten oder verwandten Verbindungen umsetzt, das Stickstoffatom mit einem geeigneten Essigsäurederivat alkyliert oder acyliert und schliesslich den schwefelhaltigen Fünfring schliesst. Zum Aufbau von Carbapenemen wird beispielsweise eine Verbindung der Formel I oder ein geschütztes Derivat einer solchen Verbindung mit einem geeignet substituierten Enolsilylether, Zinnenolat, Borenolat, Tetraallyzinn oder einer verwandten Verbindung umgesetzt und die erhaltenen Produkte entsprechend weiterverarbeitet.

Wesentliche Kriterien für antibiotische Wirkung von Penemen und Carbapenemen sind nicht nur Art und Stellung, sondern auch die räumliche Anordnung der Substituenten. Das Diastereomere der Formel Ia weist eine günstige räumliche Anordnung der Hydroxy-, Hydroxyethyl- und Acetoxygruppe auf, um als Ausgangsmaterial für antibiotisch wirksame β-Lactam-Antibiotika zu dienen. Aber auch andere Diastereomere sind für die Weiterverarbeitung geeignet: Die an C(4) epimere cis-Verbindung (4S) ergibt bei der Substitutionsreaktion mit Schwefel- und Kohlenstoff-Nukleophilen unter geeigneten Reaktionsbedingungen ebenfalls die in der Verbindung der Formel Ia schon vorhandene trans-Anordnung der Substituenten am Azetidinon-Ring.

Die Herstellung von Verbindung der Formel I ist bekannt. In EP 78026 und Tetrahedron Letters 23, 2293-2296 (1982) ist beispielsweise ein Verfahren beschrieben, bei dem die Verbindung der Formel Ia aus L-Asparaginsäure über Acylierung oder Hydroxyalkylierung des Dianions einer N-geschützten Azetidinon-4-carbonsäure und nachfolgende oxidative Decarboxylierung hergestellt wird. EP 106652 beschreibt ein Verfahren, bei welchem eine Schiff'sche Base von Glyoxylsäure mit Diketen umgesetzt, die Acetylgruppe in der Seitenkette zu Hydroxyethyl reduziert und eine Enantiomerentrennung über diastereomere Ester vorgenommen wird. In EP 171064 wird ein Prozess beschrieben, bei dem ein 4-Ethynylazetidinon durch Cycloaddition aufgebaut wird und anschliessend der Ethynylrest in Acetyl und schliesslich durch Baeyer-Villiger-Reaktion in Acetoxy umgewandelt wird. EP 181831 beschreibt ein Verfahren, bei welchem ein α,β-Epoxybutyryl-acetonylamid mit Base cyclisiert und das gebildete 4-Acetyl-3-hydroxyethyl-azetidinon zur 4-Acetoxy-Verbindung aufoxidiert wird.

Beschreibung des Verfahrens

Die Erfindung betrifft ein Verfahren zur Herstellung von Verbindungen der Formel

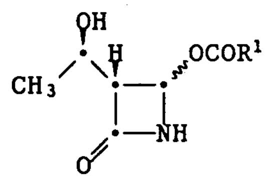

(I),

worin $R^1$ $C_1$-$C_7$-Niederalkyl oder Aryl, das 6 bis 14 C-Atome enthält und durch $C_1$-$C_7$-Niederalkyl, Hydroxy, $C_1$-$C_7$-Niederalkoxy, $C_1$-$C_8$-Niederalkanoyloxy, Halogen oder Nitro substituiert sein kann, bedeutet, dadurch gekennzeichnet, dass man eine Verbindung der Formel

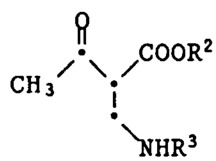

(II),

worin $R^2$ Wasserstoff oder einen veresternden Rest und $R^3$ Wasserstoff oder einen Acylrest aus der Gruppe $C_1$-$C_8$-Niederalkanoyl, $C_3$-$C_8$-Cycloalkylcarbonyl, $C_6$-$C_{14}$-Arylcarbonyl, $C_1$-$C_7$-Niederalkoxycarbonyl, $C_6$-$C_{14}$-Aryloxycarbonyl und $C_6$-$C_{14}$-Aryl-$C_1$-$C_7$-niederalkoxycarbonyl bedeuten, oder ein Salz davon mit einem enantioselektiven Reduktionsmittel reduziert, eine erhältliche Verbindung der Formel

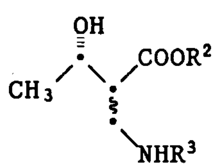

(IIIa),

falls $R^2$ und/oder $R^3$ Wasserstoff bedeuten, in beliebiger Reihenfolge verestert beziehungsweise mit einem den Acylrest $R^3$ einführenden Reagens acyliert, und die Verbindung der Formel IIIa, worin $R^2$ einen veresternden Rest und $R^3$ einen Acylrest $R^3$ bedeuten, oder ein Salz davon mit einem die Hydroxygruppe aktivierenden Mittel cyclisiert, eine erhältliche Verbindung der Formel

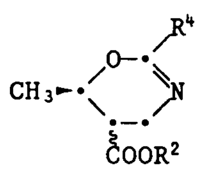

(IV),

worin $R^2$ einen veresternden Rest und $R^4$ $C_1$-$C_7$-Niederalkyl, $C_3$-$C_8$-Cycloalkyl, $C_6$-$C_{14}$-Aryl, $C_1$-$C_7$-Niederalkoxy, $C_6$-$C_{14}$-Aryloxy oder $C_6$-$C_{14}$-Arylniederalkoxy, worin Aryl wie unter $R^1$ angegeben substituiert sein kann, bedeuten, mit Base isomerisiert und ein erhältliches Diastereomeres der Formel

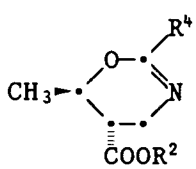

(IVa)

hydrolysiert, die erhältliche Verbindung der Formel

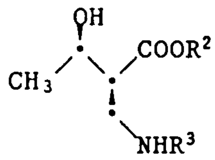

(IIIb),

worin $R^2$ und $R^3$ Wasserstoff bedeuten, oder ein Salz davon mit einem wasserentziehenden Mittel cyclisiert, und die erhältliche Verbindung der Formel

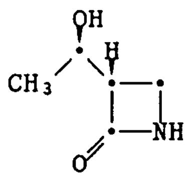

(V)

mit einem den Rest $R^1COO$- einführenden Mittel oxidiert.

Gegenüber den vorbekannten Verfahren weist das neue erfindungsgemässe Verfahren wesentliche Vorteile auf, können doch damit die Verbindungen der Formel I und deren Diastereomere mit hoher Gesamtausbeute, hoher Stereoselektivität, wenig Reaktionsschritten und einfachen und preisgünstigen Reagenzien hergestellt werden. Ueberraschenderweise ist es möglich, ausgehend von leicht zugänglichen, billigen Verbindungen der Formel II mit einem enantioselektiven Reduktionsschritt und einfachen Folgereaktionen zu enantiomerenreinen Diastereomeren der Formel I mit drei chiralen Kohlenstoffatomen zu gelangen. Es ist auch überraschend, dass die Reaktionssequenz zur Herstellung der Verbindung I, die einen empfindlichen $\beta$-Lactamring enthält, keine Schutzgruppe benötigt.

Die in der Definition von Substituenten verwendeten allgemeinen Ausdrücke und Bezeichnungen haben vorzugsweise die folgenden Bedeutungen:

Nieder, z.B. in Niederalkyl, Niederalkoxy oder Niederalkanoyl, bedeutet, dass die so bezeichneten Reste und Gruppen, wo nichts anderes angegeben, 1 bis 7 C-Atome und bevorzugt 1 bis 4 C-Atome enthalten.

Ein veresternder Rest $R^2$ ist beispielsweise Niederalkyl, Cycloalkyl oder Arylniederalkyl, vorzugsweise Niederalkyl.

Der Acylrest $R^3$ ist vorzugsweise Niederalkanoyl oder $C_6$-$C_{14}$-Arylcarbonyl und der Rest $R^4$ entsprechend Niederalkyl beziehungsweise $C_6$-$C_{14}$-Aryl.

Niederalkyl $R^1$, $R^2$ oder $R^4$ hat 1 bis 7 C-Atome und ist z.B. Methyl, Ethyl, n-Propyl, Isopropyl, n-Butyl oder tert-Butyl.

Aryl $R^1$ oder $R^4$ hat 6 bis 14, vorzugsweise 6 bis 10, C-Atome und ist beispielsweise unsubstituiertes oder substituiertes Phenyl, 1-Naphthyl oder 2-Naphthyl. Als Substituenten kommen beispielsweise Niederalkyl, z.B. Methyl, Hydroxy, Niederalkoxy, z.B. Methoxy, $C_1$-$C_8$-Niederalkanoyloxy, z.B. Acetoxy, Halogen, z.B. Chlor oder Brom, oder Nitro in Frage. Die Substituenten können in 2-, 3- oder 4-Stellung des Phenylringes stehen und auch mehrfach vorkommen, z.B. wie in 4-Methylphenyl, 3,5-Dimethylphenyl, 4-Methoxyphenyl, 4-Chlorphenyl, 4-Nitrophenyl, 2,4-Dinitrophenyl oder 3,5-Dinitrophenyl.

Cycloalkyl $R^2$ oder $R^4$ enthält 3 bis 8, vorzugsweise 3 bis 6, C-Atome und ist z.B. Cyclopropyl, Cyclopentyl oder Cyclohexyl.

In Arylniederalkyl $R^2$ hat Niederalkyl vorzugsweise 1 bis 4 C-Atome und ist z.B. Methyl oder Ethyl. Aryl in Arylniederalkyl $R^2$ hat die unter Aryl $R^1$ genannten Bedeutungen und kann auch mehrfach vorkommen, beispielsweise zwei- oder dreimal. Beispiele für Arylniederalkyl $R^2$ sind Benzyl, 4-Methylbenzyl, 4-Methoxybenzyl, 4-Nitrobenzyl, 2-Phenylethyl, Diphenylmethyl, Di(4-methoxyphenyl)methyl, Trityl, 1-Naphthylmethyl und 2-Naphthylmethyl.

Niederalkanoyl $R^3$ hat 1 bis 8, vorzugsweise 2 bis 5, C-Atome und ist z.B. Acetyl, Propionyl, Butyryl oder Pivaloyl.

Cycloalkylcarbonyl $R^3$ enthält beispielsweise 4 bis 9, vorzugsweise 4 bis 7, C-Atome, d.h. 3-8, vorzugsweise 3-6, Ringkohlenstoffatome und ist z.B. Cyclopropylcarbonyl, Cyclopentylcarbonyl oder Cyclohexylcarbonyl.

In Arylcarbonyl $R^3$ hat Aryl 6 bis 14 C-Atome und hat die unter Aryl $R^1$ genannten Bedeutungen. Arylcarbonyl $R^3$ ist beispielsweise Benzoyl, p-Toluyl, 4-Methoxybenzoyl, 3,5-Dinitrobenzoyl, 1-Naphthylcarbonyl oder 2-Naphthylcarbonyl.

In Niederalkoxycarbonyl $R^3$ hat Niederalkoxy 1 bis 7 C-Atome und ist z.B. Methoxy, Ethoxy, n-Propoxy, n-Butoxy oder Isobutoxy.

In Aryloxycarbonyl $R^3$ hat Aryloxy 6 bis 14, vorzugsweise 6 bis 10, C-Atome und ist beispielsweise unsubstituiertes oder substituiertes Phenoxy, 1-Naphthoxy oder 2-Naphthoxy. Als Substituenten von Phenoxy kommen die weiter oben unter Aryl $R^1$ genannten Reste in Frage, beispielsweise Niederalkyl, z.B. Methyl, Niederalkoxy, z.B. Methoxy, Halogen, z.B. Chlor, oder Nitro.

In Arylniederalkoxycarbonyl $R^3$ besteht Arylniederalkoxy aus Niederalkoxy mit vorzugsweise 1 bis 4 C-Atomen, z.B. aus Methoxy oder Ethoxy, und Aryl mit den weiter oben unter Aryl $R^1$ genannten Bedeutungen, wobei Aryl auch mehrfach vorkommen kann, beispielsweise zwei- oder dreimal. Beispiele für solches Arylniederalkoxy sind Benzyloxy, 4-Methylbenzyloxy, 4-Methoxybenzyloxy, 4-Nitrobenzyloxy, 2-Phenylethoxy und Diphenylmethoxy.

Niederalkoxy, Aryloxy und Arylniederalkoxy $R^4$ haben die oben unter den entsprechenden Resten Niederalkoxycarbonyl, Aryloxycarbonyl und Arylniederalkoxycarbonyl $R^3$ genannten Bedeutungen.

Salze sind beispielsweise Säureadditionssalze der Aminogruppe in Verbindungen der Formel II, III, IIIa oder IIIb, z.B. mit anorganischen Säuren, z.B. Salzsäure, Schwefelsäure, Salpetersäure oder Phosphorsäure, oder mit organischen Carbon- oder Sulfonsäuren, z.B. mit Essigsäure, Chloressigsäure, Trichloressigsäure, Trifluoressigsäure, Propionsäure, Glykolsäure, Fumarsäure, Benzoesäure, Methansulfonsäure, Trifluormethansulfonsäure, Ethansulfonsäure, Campher-10-sulfonsäure, Benzolsulfonsäure, Toluolsulfonsäure, 4-Nitrobenzolsulfonsäure, 2,4-Dinitrobenzolsulfonsäure oder Naphthalin-2-sulfonsäure. Weitere Säureadditionssalze werden vom Stickstoff der Imidestergruppe in 1,3-Oxazinen der Formel IV oder IVa mit starken Säuren, beispielsweise Mineralsäuren, z.B. Salzsäure oder Schwefelsäure, oder mit organischen Sulfonsäuren, z.B. den oben genannten Sulfonsäuren, gebildet.

Carbonsäuren der Formeln II, III, IIIa oder IIIb können Alkalimetall-, z.B. Natrium- oder Kaliumsalze, bilden, ferner Erdalkalimetallsalze, z.B. Magnesium- oder Calciumsalze, Schwermetallsalze, z.B. Kupfer-, Blei- oder Zinksalze, Ammoniumsalze, Salze mit organischen Aminen, z.B. mit gegebenenfalls substituierten Mono-, Di-oder Trialkylaminen, z.B. mit Cyclohexylamin, Diethylamin, Cyclohexylethylamin, Dibutylamin, Trimethylamin, Triethylamin oder Tri-(2-hydroxyethyl)-amin, oder mit tetrasubstituierten organischen Ammoniumionen, z.B. mit Tetramethylammonium, Tetraethylammonium oder Tetrabutylammonium.

Verbindungen der Formel II, III, IIIa und IIIb, in denen $R^2$ und $R^3$ Wasserstoff bedeuten, können auch innere Salze bilden.

Eine Verbindung der Formel I oder Ia besitzt drei voneinander unabhängige chirale Kohlenstoffatome, nämlich Kohlenstoffatome 3 und 4 des $\beta$-Lactamringes und das die Hydroxygruppe tragende Kohlenstoffatom 1′ der Hydroxyethyl-Seitenkette. Die Formel I umfasst zwei diastereomere Verbindungen, nämlich das (3R,4R,1′R)-Isomere, z.B. die Verbindung der Formel Ia, und das (3R,4S,1′R)-Isomere.

Die Verbindungen der Formeln III, IIIa, IIIb, IV und IVa besitzen zwei voneinander unabhängige chirale Kohlenstoffatome. Die Formel

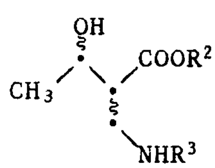

(III)

umfasst alle 4 möglichen Diastereomeren. Die Formel IIIa umfasst zwei dieser Diastereomere, nämlilch das (2R,3S)-Isomere und das (2S,3S)-Isomere. Eine Verbindung der Formel IIIb weist hingegen die (2S,3R)-Konfiguration auf. Die Formel IV umfasst ebenfalls zwei diastereomere Verbindungen, nämlich das (5R,6R)-Isomere und das (5S,6R)-Isomere, d.h. die Verbindung der Formel IVa.

Die Verbindung der Formel V ist konfigurativ einheitlich und stellt das (3S,1′R)-Isomere dar.

Im Zusammenhang mit der vorliegenden Beschreibung heisst diastereomerenrein, dass das entsprechende Diastereomere zu mindestens 80 % im Gemisch mit anderen Diastereomeren vorliegt. Dieser hohe Anteil eines Diastereomeren erlaubt, durch einfach physikalische Trennmethoden, z.B. durch Umkristallisation einer erfindungsgemässen Verbindung, eines geeigneten Salzes oder eines geeigneten Derivates, den Anteil des Diastereomeren auf beispielsweise über 95 % oder so zu erhöhen, dass die andern Diastereomeren mit den üblichen analytischen Methoden nicht mehr nachgewiesen werden können. Enantiomerenrein

ist analog definiert.

Verfahrensschritte

Die Reduktion einer Verbindung der Formel II geschieht auf chemischem oder biochemischem Weg.

Ein geeignetes enantioselektives Reduktionsmittel ist ein Reduktionsmittel, das bei der Reaktion mit Verbindungen der Formel II vorwiegend Diastereomere der Formel IIIa ergibt, die die (3S)-Konfiguration aufweisen, also aus racemischem Ausgangsmaterial zu einer optisch aktiven Verbindung führt. Dabei ist es für das erfindungsgemässe Verfahren unerheblich, ob gleichzeitig auch eine Konfiguration an C(2), z.B. die (2S)-Konfiguration, bevorzugt wird.

Ein bevorzugtes Beispiel für ein biochemisches enantioselektives Reduktionsmittel ist die Bäckerhefe (Saccharomyces cerevisiae) und die in Bäckerhefe enthaltenen Oxidoreduktasen. Für die Reduktion kann Bäckerhefe irgendwelcher Herkunft verwendet werden, beispielsweise die von der American Type Culture Collection unter der Nummer ATCC 26403 erhältliche Bäckerhefe. Auch andere Hefen oder andere Pilze sind geeignet, beispielsweise Candida albicans, Geotrichum candidum, Schizosaccharomyces pombs, Saccharomyces acidfaciens, Rhodotorula rubra, Curvularia lunata, Curvularia falcata oder Mortierella ramanniana. Ebenfalls geeignet sind isolierte Oxidoreduktasen, vorzugsweise von $\beta$-Nicotinamid-adenind inucleotid-dihydrid (NADH) oder von NADH-phosphat (NADPH) abhängige Oxidoreduktasen, beispielsweise Hefe-Alkohol-Dehydrogenase, Dihydroxyaceton-Reduktase aus Mucor javanicus, $\beta$-Hydroxyacyl-CoA-Dehydrogenase aus Schweineherz, Fettsäure-Synthetase (enthaltend eines $\beta$-Keto-Reduktase-Komponente) aus Bäckerhefe, D-$\beta$-Hydroxybutyrat-Dehydrogenase aus Pseudomonas lemoignei oder Rhodopseudomonas spheroides und weitere Oxidoreduktasen.

Bei der Reduktion mit ganzen Zellen, beispielsweise mit Bäckerhefe, wird der Mikroorganismus unter physiologischen Bedingungen in einer geeigneten Vorkultur gehalten und, wenn nötig, expandiert. Die Kulturmedien sind die üblichen Medien, beispielsweise gepufferte Glucoselösung, die gewünschtenfalls, Spurenelemente enthält, mit einem pH-Wert zwischen 6 und 8. Das Substrat der Formel II wird in Konzentrationen von 1 bis 50 g pro Liter zum Kulturmedium gegeben und weitere Glucose, das die Funktion des Wasserstoffspenders übernimmt, oder andere Kohlenhydrate, ferner beispielsweise auch ein Formiat, ein Hypophosphit oder Ethanol als Wasserstoffspender, zugegeben. Die Kultur wird zwischen Raumtemperatur und 40°C, vorzugsweise bei leicht erhöhter Temperatur, z.B. zwischen 30°C und 37°C gehalten und intensiv durchmischt.

Bei der Reduktion mit isolierten Enzymen wird vorzugsweise ein weiteres Enzym beigesetzt, das in situ das für die Oxidoreduktase notwendige NADH beziehungsweise NADPH regeneriert, beispielsweise Clostridium kluyveri oder Enzyme aus C. kluyveri. Als Wasserstoffspender eignen sich dabei molekularer Wasserstoff, ein Formiat, ein Hypophosphit oder eine elektrochemische Reduktion in Gegenwart eines Viologens, z.B. Methylviologen. Es ist auch möglich, ohne weitere Enzyme NADH beziehungsweise NADPH mit z.B. Ethanol oder Formiat zu regenerieren.

Chemische enantioselektive Reduktionsmittel sind beispielsweise Wasserstoff in Gegenwart eines chiralen heterogenen Katalysators oder eines chiralen homogenen Katalysators, chirale Borane und Borhydride und chirale Aluminiumhydride.

Ein chiraler heterogener Katalysator ist beispielsweise ein mit enantiomereneiner Weinsäure und Natriumbromid modifizierter Raney-Nickel-Katalysator oder ein mit einem chiralen Amin, z.B. enantiomerenreinem 1-Phenylethylamin, modifizierter Platin-Katalysator. Geeignete chirale homogene Katalysatoren sind beispielsweise Rhodium-bisphosphin-hydrid-Komplexe, in denen das Phosphin chiral ist, z.B. enthaltend enantiomerenreines Cyclohexyl-methyl-o-methoxyphenylphosphin oder 2,3-0-isopropyliden-2,3-dihydroxy-1,4-bis (diphenylphosphino)butan (DIOP).

Geeignete chirale Borane und Borhydride sind beispielsweise enantiomerenreines Diisopinocampheylboran oder Lithium-B-isopinocampheyl-9-borabicyclo[3.3.1]nonylhydrid. Geeignete chirale Aluminiumhydride werden durch Reaktion von Lithiumaluminiumhydrid mit äquivalenten Mengen eines enantiomerenreinen, chiralen Alkohols, Amins oder Aminoalkohols, z.B. 1,1′-Di($\beta$-naphthol), o,o-Dimethyl-N-pyrrolidinylmethylanilin oder Darvonalkohol, erhalten.

Die genannten chemischen enantioselektiven Reduktionsmittel werden unter den üblichen Reaktionsbedingungen eingesetzt. Hydrierungen in Gegenwert von chiralen heterogenen oder homogenen Katalysatoren werden beispielsweise je nach Art des Katalysators bei einem Wasserstoffdruck zwischen 1 und 100 bar und Temperaturen von 0° bis 120°C in einem Lösungsmittel, beispielsweise in einem Alkohol, z.B. Ethanol, inerten einem Ester, z.B. Essigsäureethylester, oder einem Kohlenwasserstoff, z.B. Cyclohexan oder Toluol, durchgeführt. Für die Reduktion mit chiralen Boranen, Borhydriden und Aluminiumhydriden verwendet man vorzugsweise wasserfreie etherische Lösungsmittel, beispielsweise Diethylether, Tetrahydrofuran oder 1,2-Dimethoxyethan, und Temperaturen zwischen -80° und +50°C, vorzugsweise zwischen -30° und 0°C.

Ist in einer durch enantioselektive Reduktion erhältlichen Verbindung der Formel IIIa die Bedeutung von $R^2$ Wasserstoff, so muss vor dem Ringschluss zu einer Verbindung der Formel IV die freie Carboxygruppe verestert werden. Die Veresterung wird unter den üblichen Bedingungen durchgeführt, beispielsweise durch Reaktion mit einem Ueberschuss des entsprechenden Alkohols $R^2OH$, z.B. eines Niederalkanols, Cycloalkanols oder Arylniederalkanols, in Gegenwart einer Säure, beispielsweise einer möglichst wasserfreien Mineralsäure, z.B. Chlorwasserstoff, konzentrierte Schwefelsäure, Polyphosphorsäure oder Phosphorpentoxid, einer organischen Sulfonsäure, z.B. p-Toluolsulfonsäure, eines sauren Ionenaustauschers oder einer Lewis-Säure, z.B. Bortrifluoridetherat, gewünschtenfalls in Gegenwart eines wasserentziehenden Mittels, beispielsweise Molekularsieb, oder unter Entfernung des Reaktionswassers durch azeotrope Destillation, beispielsweise mit einem Halogenkohlenwasserstoff, z.B. Methylenchlorid, Chloroform oder Tetrachlorkohlenstoff, oder einem aromatischen Kohlenwasserstoff, z.B. Benzol oder Toluol. Die saure Veresterung wird beispielsweise bei Temperaturen zwischen 0° und dem Siedepunkt des Alkohols $R^2OH$ oder des Azeotrops mit den genannten Schlepper-Lösungsmitteln durchgeführt. Eine Veresterung kann auch mit einem Alkylierungsmittel erreicht werden, z.B. mit Diazomethan in etherischer Lösung oder mit Benzylchlorid un dem Natrium- oder vorzugsweise Cäsiumsalz der Verbindung der Formel IIIa.

Ist in einer durch enantioselektive Reduktion erhältlichen Verbindung der Formel IIIa die Bedeutung von $R^3$ Wasserstoff, so muss vor dem Ringschluss zu einer Verbindung der Formel IV die freie Aminogruppe acyliert werden. Die Acylierung erfolgt unter den üblichen Reaktionsbedingungen, beispielsweise unter Verwendung des der Acylgruppe $R^3$ entsprechenden Carbonsäureanhydrids oder Carbonsäurehalogenids, z.B. Carbonsäurechlorids, oder des der Acylgruppe $R^3$ entsprechenden Kohlensäurehalbesterderivates, beispielsweise Halogenameisensäureesters, z.B. Chlorameisensäureesters, gegebenenfalls unter Zugabe einer Säure, z.B. p-Toluolsulfonsäure, oder einer Base, z.B. eines tertiären Amins, z.B. Triethylamin, Dimethylbenzylamin oder N-Methylmorpholin, eines Amidins, z.B. 1,5-Diazabicyclo[4.3.0]non-5-en oder 1,8-Diazabicyclo[5.4.0]undec-7-en, einer Pyridinbase, z.B. Pyridin, Lutidin oder 4-Dimethylaminopyridin, oder eines Alkali- oder Erdalkalimetallcarbonats, z.B. Natrium-, Kalium- oder Calciumcarbonat, in einem inerten Lösungsmittel, beispielsweise in einem Ether, z.B. Diethylether, Tetrahydrofuran oder Dioxan, einem Halogenkohlenwasserstoff, z.B. Methylenchlorid, einem Ester, z.B. Essigsäureethylester, einem Amid, z.B. Dimethylformamid oder N-Methylpyrrolidon, oder in einem Nitril, z.B. Acetonitril, bei Temperaturen zwischen -20°C und dem Siedepunkt des Lösungsmittels, vorzugsweise zwischen 0° und 50°C. Ein Ueberschuss des Acylierungsmittels ist zu vermeiden, damit nicht gleichzeitig die Hydroxygruppe der Verbindung der Formel IIIa acyliert wird.

Die Reihenfolge der genannten Veresterungs- und Acylierungsschritte ist frei wählbar.

Eine Verbindung der Formel IIIa, worin $R^2$ ein veresternder Rest, z.B. Niederalkyl, Cycloalkyl oder Arylniederalkyl, und $R^3$ wie oben definiertes Niederalkanoyl, Cycloalkylcarbonyl oder Arylcarbonyl beziehungsweise Niederalkoxycarbonyl, Aryloxycarbonyl oder Arylniederalkoxycarbonyl, bedeuten, wird mit einem die Hydroxygruppe an C(3) aktivierenden Mittel zu einem 5,6-Dihydro-1,3,4H-Oxazin der Formel IV cyclisiert. Die Hydroxygruppe wird dabei in eine nukleofuge Abgangsgruppe umgewandelt und intramolekular durch den Sauerstoff der Carbonylgruppe des Restes $R^3$ unter Inversion substituiert.

Geeignete die Hydroxygruppe aktivierende Mittel sind beispielsweise die üblicherweise zur Herstellung von Halogeniden aus Alkoholen verwendeten Halogenierungsmittel, beispielsweise Phosphorhalogenide, z.B. Phosphorpentachlorid, Phosphortrichlorid, Phosphortribromid, Triphenylphosphindichlorid, Triphenylphosphindibromid oder äquivalente Mengen von Triphenylphosphin und Tetrachlorkohlenstoff, ferner reaktive organische Säurehalogenide, beispielsweise Phosgen, Oxalkylchlorid, Cyanurchlorid oder Dimethylformamidiniumchlorid, oder insbesondere Thionylchlorid. Mit diesen Halogenierungsmitteln wird die Hydroxygruppe an C(3) in einen aktivierten anorganischen oder organischen Ester umgewandelt, der dann aber nicht wie üblich mit einem Halogenidion abreagiert, sondern intramolekular durch den Sauerstoff der Carbonylgruppe substituiert wird. Die Reaktion wird ohne Lösungsmittel oder vorzugsweise in einem inerten Lösungsmittel, beispielsweise in einem Kohlenwasserstoff, z.B. Toluol, einem Chlorkohlenwasserstoff, z.B. Methylenchlorid, einem Ester, z.B. Essigsäureethylester, oder einem Ether, z.B. Diethylether oder Dioxan, bei Temperaturen zwischen 0° und 150°C, z.B. dem Siedepunkt des Lösungsmittel oder bei Raumtemperatur durchgeführt. Gewünschtenfalls wird eine Base zugesetzt, die den frei werdenden Halogenwasserstoff bindet, biespielsweise ein tertiäres Amin, z.B. Triethylamin, Dimethylanilin oder N-Methylmorpholin, oder eine Pyridinbase, z.B. Pyridin, ferner gewünschtenfalls katalytische Mengen eines Amids, z.B. Dimethylformamid oder Hexamethylphosphorsäuretriamid, oder einer Lewis-Säure, z.B. Zinkchlorid oder Aluminiumchlorid. Die Reaktion wird vorzugsweise mit äquivalenten Mengen oder mit einem geringen Ueberschuss, z.B. 1,05 bis 1,5 Aequivalenten, des Halogenierungsmittels durchgeführt.

Weitere geeignete, die Hydroxygruppe aktivierende Mittel sind Reagentien zur Herstellung von Sulfonaten aus Alkoholen, beispielsweise Sulfonsäurehalogenide, z.B. Methansulfonsäurechlorid, Benzolsulfonsäurechlorid, p-Toluolsulfonsäurechlorid, p-Nitrobenzolsulfonsäurechlorid oder 2,4-Dinitrobenzolsulfonsäurechlorid, oder Sulfonsäureanhydride, z.B. Trifluormethansulfonsäureanhydrid. Diese Reagentien werden beispielsweise in den oben bei den Halogenierungsmitteln genannten inerten Lösungsmitteln, z.B. Methylenchlorid oder Diethylether, in Gegenwart einer der genannten Basen, z.B. Triethylamin, bei Temperaturen zwischen -30° und 50°C, z.B. zwischen 0°C und Raumtemperatur, eingesetzt.

Weitere die Hydroxygruppe aktivierende Mittel sind reaktive Carbonylderivate, beispielsweise Carbonylbisimidazol oder Di-2-pyridylcarbonat, und Kombinationen von Azoverbindungen mit Phosphinen, beispielsweise Azodicarbonsäurediethylester und Triphenylphosphin oder 4-Methyl-1,2,4-triazolidin-3,5-dion und Triphenylphosphin. Die genannten Reagentien werden vorzugsweise in polaren inerten Lösungsmitteln, z.B. Acetonitril, Tetrahydrofuran oder 1,2-Dimethoxyethan, bei Temperaturen zwischen -30° und +50°C, z.B. zwischen 0°C und Raumtemperatur, verwendet.

In einer Verbindung der Formel IV, worin $R^2$ einen veresternden Rest, z.B. Niederalkyl, Cycloalkyl oder Arylniederalkyl, und $R^4$ wie oben definiertes Niederalkyl, Cycloalkyl oder Aryl beziehungsweise Niederalkoxy, Aryloxy oder Arylniederalkoxy bedeuten, kann durch Behandlung mit geeigneten Basen eine Umwandlung des (5R,6R)-Diastereomeren oder von Mischungen der (5R,6R)- und (5S,6R)-Diastereomeren in die diastereomerenreine Verbindung der Formel IVa mit (5S,6R)-Konfiguration, d.h. trans-Anordnung der Substituenten $CH_3$ und $COOR^2$ am Sechsring, erreicht werden. Geeignete Basen für eine solche Isomerisierung sind beispielsweise tertiäre Amine, z.B. Triethylamin oder Tributylamin, Pyridin oder Pyridinderivate, z.B. Lutidin, Amidinbasen, z.B. 1,5-Diazabicyclo[4.3.0]non-5-en oder 1,8-Diazabicyclo[5.4.0]undec-7-en, Alkalimetallsalze von sterisch gehinderten, sekundären Aminen, z.B. Lithiumdiisopropylamid oder Lithiumhexamethyldisilazid, oder basische Alkali-und Erdalkalimetallsalze, z.B. Natrium- oder Kaliumcarbonat oder Kaliumfluorid. Die Isomerisierung wird beispielsweise in polaren, wasserfreien Lösungsmitteln, z.B. Ethanol, tert-Butanol, Tetrahydrofuran, Dioxan oder Acetonitril, bei Temperaturen zwischen 50° und 100°C, oder in unpolaren, wasserfreien Lösungsmitteln, z.B. aromatischen Kohlenwasserstoffen, z.B. Toluol, Chlorbenzol oder Dichlorbenzol, bei Temperaturen zwischen 80° und 150°C, beispielsweise beim Siedepunkt des Lösungsmittels, durchgeführt.

Im nächsten Schritt wird in einer Verbindung der Formel IVa der 1,3-Oxazinring gespalten und gleichzeitig die Acylaminogruppe und die Esterfunktion hydrolysiert. Die Hydrolyse wird vorzugsweise mit Säuren durchgeführt, ist aber auch mit Basen möglich.

Geeignete Säuren sind beispielsweise Mineralsäuren, z.B. Salzsäure, Schwefelsäure oder Phosphorsäure, oder starke organische Säuren, z.B. Alkan- oder Arylsulfonsäuren, z.B. Methansulfonsäure, Trifluormethansulfonsäure, Benzolsulfonsäure, p-Toluolsulfonsäure oder p-Nitrobenzolsulfonsäure, vorzugsweise in wässriger Lösung, beispielsweise in Wasser oder in Mischungen von Wasser und organischen Lösungsmitteln, z.B. Ethanol, Dioxan, Ethylenglykol-monomethylether oder -monobutylether, bei Temperaturen zwischen 80° und 150°C, vorzugsweise um 100°C.

Geeignete Basen sind Metallhydroxide, beispielsweise Alkalimetallhydroxide, z.B. Lithium-, Natrium- oder Kaliumhydroxid, Alkalimetallcarbonate, z.B. Natrium- oder Kaliumcarbonat, oder tetrasubstituierte Ammoniumhydroxide, z.B. Benzyltrimethylammoniumhydroxid, vorzugsweise in Wasser oder den obengenannten Mischungen von Wasser und organischen Lösungsmitteln bei Temperaturen zwischen 50° und 120°C, z.B. um 80°C. Der basischen Hydrolyse kann zur Beschleunigung Wasserstoffperoxid, z.B. als 30 % wässrige Lösung, zugesetzt werden.

Die bei der Hydrolyse erhältliche Verbindung der Formel IIIb, worin $R^2$ und $R^3$ Wasserstoff bedeuten, wird mit den üblichen wasserentziehenden Mitteln zum $\beta$-Lactam der Formel V cyclisiert. Geeignete wasserentziehende Mittel sind Acetylchlorid, Thionylchlorid oder Phosphortrichlorid, Acetanhydrid, Triethylaluminium, Triisobutylaluminium oder dergleichen, vorzugsweise jedoch ein Carbodiimid, z.B. Dicyclohexylcarbodiimid oder Diisopropylcarbodiimid, 2-Chlor-1-methylpyridiniumiodid, oder 2,2′-Dipyridyldisulfid in Gegenwart eines Phosphins, z.B. Triphenylphosphin. Carbodiimide werden vorzugsweise in Gegenwert äquivalenter Mengen eines tertiären Amins, z.B. Triethylamin, oder einer Pyridinbase, z.B. Pyridin, in einem inerten polaren Lösungsmittel, z.B. Acetonitril, Dimethylformamid, Nitromethan, Tetrahydrofuran oder Dioxan, bei Temperaturen zwischen 25° und 80°C, z.B. um 50°C, eingesetzt. Bei der Reaktion mit 2-Chlor-1-methylpyridiniumiodid wird eine äquivalente Menge eines tertiären Amins, z.B. Triethylamin, zugegeben und die Kondensation bei Temperaturen zwischen 0° und 50°C, z.B. um Raumtemperatur, in einem inerten Lösungsmittel, z.B. Methylenchlorid, bewerkstelligt. Die Kondensation mit 2,2′-Dipyridyldisulfid und Triphenylphosphin wird in einem der genannten polaren Lösungsmittel, vorzugsweise in Acetonitril, bei Temperaturen zwischen 50° und 85°C, z.B. beim Siedepunkt von Acetonitril, vorgenommen. Anstelle von 2,2′-Dipyridyldisulfid kann auch 2-Mercaptopyridin in katalytischen Mengen und eine äquivalente Menge eines

milden Oxidationsmittels, bei spielsweise Manganoxid, eingesetzt werden.

Weiter geeignet als wasserentziehende Mittel sind die in der Peptidchemie zur Kondensation von α-Aminosäuren verwendeten Reagentien, beispielsweise Bis(2-oxo-3-oxazolidinyl)phosphinsäurechlorid, 1-Benztriazolyloxy-tris(dimethylamino)phosphonium-hexafluorophosphat oder 2-Ethoxy-1-ethoxycarbonyl-1,2-dihydrochinolin.

Die Verbindung der Formel V wird mit einem den Rest $R^1COO$- einführenden Mittel zu einer Verbindung der Formel I oxidiert. Die Oxidation erfolgt chemisch oder vorzugsweise elektrochemisch.

Chemische, den Rest $R^1COO$- einführende Oxidationsmittel sind beispielsweise die entsprechenden Acylperoxide, z.B. Acetyl-tert-butylperoxid, Propionyl-tert-butylperoxid, Benzoyl-tert-butylperoxid oder Dibenzoylperoxid. Vorzugsweise werden die tert-Butylperoxide mit katalytischen Mengen eines Kupferf(I)-salzes, z.B. Kupfer(I)-chlorid, -bromid oder -2-ethylhexanoat, in einem inerten Lösungsmittel, z.B. Benzol, Petrolether, Acetonitril oder Acetonitril/Eisessig, umgesetzt. Bei der Reaktion mit Dibenzoylperoxid wird kein Katalysator benötigt. Weitere, den Rest Acetyl einführende Oxidationsmittel sind beispielsweise Bleitetraacetat und Quecksilberdiacetat.

Zur elektrochemischen Oxidation verwendet man eine Monozelle oder eine geteilte Zelle mit einem mechanischen Diaphragma, z.B. aus porösem Ton, Glas oder Kunststoff, z.B. Polyvinylchlorid oder Polypropylen, oder mit einem Ionenaustauscher-Diaphragma, beispielsweise wie unter dem Handelsnamen Nafion erhältlich, und Elektroden aus Edelmetall, beispielsweise Platin, Titanlegierungen, z.B. Titan-Iridium oder Titan-Tantal, ferner Nickel, Bleidioxid, Glaskohlenstoff und/oder Graphit. Die elektrochemische oxidative Acylierung wird in der dem Rest $R^1$ entsprechenden Carbonsäure, z.B. Essigsäure oder Propionsäure, oder in Gemischen dieser Säure und inerten organischen Lösungsmitteln, beispielsweise Acetonitril, Dioxan oder Dimethylformamid, oder tertiären Aminen, z.B. Triethylamin, beispielsweise in Essigsäure/Acetonitril-, Essigsäure/Triethylamin-, Propionsäure/Acetonitril- oder Benzoesäure/Acetonitril-Gemischen, durchgeführt, wobei Leitsalze, beispielsweise Lithium-, Natrium-, Kalium- oder Tetraalkylammoniumsalze, z.B. Lithium-, Natrium-, Tetraethylammonium- oder Tetrabutylammonium-tetrafluoroborat, -acetat oder -nitrat, zugesetzt werden. Geeignete Stromdichten für die Elektrolyse liegen zwischen 10 und 400 mA/cm$^2$, beispielsweise um 50 mA/cm$^2$, bei Temperaturen zwischen 0° und 50°C, vorzugsweise zwischen Raumtemperatur und 30°C.

Sowohl die chemische oxidative Acylierung als auch die elektrochemische oxidative Acylierung ergeben bevorzugt eine Verbindung der Formel I, worin die Hydroxyethylgruppe an C(3) und die Acyloxygruppe an C(4) trans zueinander angeordnet sind, also das Diastereomere mit der (3R,4R,1′R)-Konfiguration.

Salze der genannten Verbindungen erhält man in üblicher Weise. Säureadditionssalze der Aminogruppe in Verbindungen der Formel II, III, IIIa oder IIIb oder des Stickstoffatoms der Imidestergruppe in Verbindungen der Formel IV oder IVa werden beispielsweise durch Behandeln mit einer Säure oder einem geeigneten Anionenaustauscherreagens gebildet, wobei man vorzugsweise stöchiometrische Mengen oder nur einen kleinen Ueberschuss des salzbildenden Mittels verwendet. Innere Salze, z.B. von Verbindungen der Formel II, III IIIa oder IIIb, welche eine freie Carboxygruppe und eine freie Aminogruppe enthalten, können z.B. durch Neutralisieren von Salzen, wie Säureadditionssalzen, auf den isoelektrischen Punkt, z.B. mit Basen, oder durch Behandeln mit Ionenaustauschern oder Epoxiden, z.B. Propylenoxid, gebildet werden. Salze von Verbindungen der Formeln II, III, IIIa oder IIIb mit Carboxygruppen kann man z.B. durch Behandeln mit Metallverbindungen, wie Alkalimetallsalzen von geeigneten organischen Carbonsäuren, z.B. dem Natriumsalz der 2-Ethylhexansäure, mit anorganischen Alkali- oder Erdalkalimetallsalzen, z.B. Natriumhydrogencarbonat, einer stöchiometrischen Menge oder einem kleinen Ueberschuss an Alkalimetallhydroxid, z.B. Lithium-, Natrium- oder Kaliumhydroxid, oder mit Ammoniak oder einem geeigneten organische Amin bilden.

Salze können in üblicher Weise in die freien Verbindungen übergeführt werden, Metall- und Ammoniumsalze z.B. durch Behandeln mit geeigneten Säuren, Säureadditionssalze z.B. durch Behandeln mit einem geeigneten basischen Mittel.

Das Verfahren der Erfindung umfasst auch die diejenigen Ausführungsformen, bei denen Zwischenprodukte isoliert und die restlichen Verfahrensschritte mit diesen durchgeführt werden, Ausgangsstoffe und Reagenzien in situ hergestellt werden und/oder Zwischen- und Endprodukte ohne Isolierung weiterverarbeitet werden, ferner die Ausführungsform, bei der eine Verbindung der Formel II direkt zu einer diastereomerenreinen Verbindung der Formel IIIb reduziert wird und ein Ringschluss zum Oxazin der Formel IV, Isomerisierung zu IVa und Rückspaltung zur diastereomerenreinen Verbindung der Formel IIIb überflüssig ist.

Insbesondere betrifft die Erfindung auch die im folgenden genannten neuen Verfahrensschritte unter den genannten bevorzugten Reaktionsbedingungen als solche oder als Teil eines gesamten Verfahrens zur Herstellung der Verbindungen der Formel I.

Bevorzugt ist ein Verfahren zur Herstellung von Verbindungen der Formel I, worin $R^1$ die obengenannte Bedeutung hat, dadurch gekennzeichnet, dass die Verbindung der Formel V elektrochemisch in Gegenwart einer Säure $R^1COOH$ bei Stromdichten zwischen 10 und 400 $mA/cm^2$ und Temperaturen zwischen 0° und 50°C oxidativ acyliert wird. Ganz besonders bevorzugt ist der genannte Verfahrensschritt zur Herstellung einer Verbindung der Formel Ia, worin $R^1$ Methyl bedeutet.

Bevorzugt ist ferner ein Verfahren zur Herstellung von Verbindungen der Formel IIIa, worin $R^2$ Wasserstoff oder einen veresternden Rest, z.B. wie oben definiertes Niederalkyl, Cycloalkyl oder Arylniederalkyl, und $R^3$ Wasserstoff oder wie oben definiertes Niederalkanoyl, Cycloalkylcarbonyl oder Arylcarbonyl beziehungsweise Niederalkoxycarbonyl, Aryloxycarbonyl oder Arylniederalkoxycarbonyl, bedeuten, dadurch gekennzeichnet, dass man eine Verbindung der Formel II, worin $R^2$ und $R^3$ die genannten Bedeutungen haben, oder ein Salz davon mit Bäckerhefe reduziert. Ganz besonders bevorzugt ist der genannte Verfahrensschritt zur Reduktion von Verbindungen der Formel II, worin $R^2$ Wasserstoff oder Niederalkyl und $R^3$ Niederalkanoyl oder Arylcarbonyl bedeuten.

In erster Linie betrifft die Erfindung die in den Beispielen beschriebenen Verfahren.

## Weiterverarbeitung

Eine Verbindung der Formel I und insbesondere das reine Diastereomere der Formel Ia kann nach einem der vielen im Stand der Technik bekannten Verfahren in wertvolle Endprodukte umgewandelt werden. Die meisten dieser Verfahren benötigen dazu ein Derivat der Verbindung der Formel I, worin die Hydroxygruppe und/oder das $\beta$-Lactam-Stickstoffatom durch eine Schutzgruppe geschützt sind. Solche Schutzgruppen lassen sich leicht einführen, beispielsweise wie in Standardwerken über Schutzgruppen beschrieben.

Vorteilhafterweise wird aber eine Verbindung der Formel I ohne Schutzgruppen weiterverarbeitet, um zusätzliche Reaktionsschritte zu vermeiden. Beispielsweise kann die Verbindung der Formel Ia, wie in der Europäischen Patentanmeldung EP 215 739 beschrieben, mit der am Stickstoffatom geschützten $\alpha$-Aminothiocarbonsäure N-Allyloxycarbonyl-thioglycin umgesetzt und in wenigen Stufen zum hochwirksamen Antibiotikum (5R,6S,1'R)-2-Aminomethyl-6-(1'-hydroxyethyl)-2-penem-3-carbonsäure verarbeitet werden. Die wertvollen Eigenschaften des genannten Endprodukts und seine Verwendung sind beispielsweise in der Deutschen Patentanmeldung DE 34 31 980 beschrieben.

## Zwischenprodukte

Neue Zwischenprodukte und Verfahren zu ihrer Herstellung sind ebenfalls Gegenstand der Erfindung.

Insbesondere betrifft die Erfindung Verbindungen der Formel III, worin $R^2$ Wasserstoff oder einen veresternden Rest, beispielsweise Niederalkyl, z.B. Methyl oder Ethyl, Cycloalkyl, z.B. Cyclohexyl, oder Arylniederalkyl, z.B. Benzyl, und $R^3$ Wasserstoff oder wie oben definiertes Niederalkanoyl, z.B. Acetyl oder Propionyl, Cycloalkylcarbonyl, z.B. Cyclohexylcarbonyl, oder Arylcarbonyl, z.B. Benzoyl, beziehungsweise Niederalkoxycarbonyl, z.B. Ethoxycarbonyl oder n-Butoxycarbonyl, Aryloxycarbonyl, z.B. Phenoxycarbonyl, oder Arylniederalkoxycarbonyl, z.B. Benzyloxycarbonyl, bedeuten, deren reine Diastereomere, Gemische zweier Diastereomere und Salze davon.

Besonders betrifft die Erfindung Verbindungen der Formel III, worin $R^2$ Wasserstoff oder Niederalkyl, z.B. Methyl oder Ethyl, und $R^3$ Wasserstoff, Niederalkanoyl, z.B. Acetyl, oder Arylcarbonyl, z.B. Benzoyl, bedeuten, deren reine Diastereomere, z.B. das Diastereomere der Formel IIIb, Gemische zweier Diastereomere, z.B. der Formel IIIa, und Salze davon.

Hauptsächlich betrifft die Erfindung Verbindungen der Formel IIIa, worin $R^2$ Wasserstoff oder Niederalkyl, z.B. Methyl oder Ethyl, und $R^3$ Niederalkanoyl, z.B. Acetyl, oder Arylcarbonyl, z.B. Benzoyl, bedeuten, deren reine Diastereomere, und Salze davon, ferner die Verbindung der Formel IIIb, worin $R^2$ und $R^3$ Wasserstoff bedeuten, und deren Salze.

Weiter betrifft die Erfindung Verbindungen der Formel IV, worin $R^2$ einen veresternden Rest, beispielsweise Niederalkyl, z.B. Methyl oder Ethyl, Cycloalkyl, z.B. Cyclohexyl, oder Arylniederalkyl, z.B. Benzyl, und $R^4$ wie oben definiertes Niederalkyl, z.B. Methyl oder Ethyl, Cycloalkyl, z.B. Cyclohexyl, oder Aryl, z.B. Phenyl, beziehungsweise Niederalkoxy, z.B. Ethoxy oder n-Butoxy, Aryloxy, z.B. Phenoxy, oder Arylniederalkoxy, z.B. Benzyloxy, bedeuten, deren reine Diastereomere und Salze davon.

Besonders betrifft die Erfindung Verbindungen der Formel IV, worin $R^2$ Niederalkyl, z.B. Methyl oder Ethyl, und $R^4$ Niederalkyl, z.B. Methyl, oder Aryl, z.B. Phenyl, bedeuten, deren reine Diastereomere, z.B. das Diastereomere der Formel IVa, und Salze davon.

Ferner betrifft die Erfindung die Verbindung der Formel V.

In erster Linie betrifft die Erfindung die in den Beispielen genannten Zwischenprodukte.

Verfahren zur Herstellung der genannten Zwischenprodukte sind insbesondere die entsprechenden Teilverfahren des weiter vorn beschriebenen erfinderischen Gesamtverfahrens. Die Verfahren schliessen auch an sich bekannte Nachbehandlungen ein, beispielsweise die Umwandlung von erfindungsgemässen Verbindungen in andere erfindungsgemässe Verbindungen und die Umwandlung von freien Verbindungen in ihre Salze oder von Salzen in die entsprechenden freien Verbindungen oder andere Salze.

Ausgangsmaterialien der Formel II sind bekannt oder können nach bekannten Methoden hergestellt werden, beispielsweise durch Amidoalkylierung von Acetessigsäureestern mit Acylamido-chlormethan gemäss H.Böhme et.al., Chemische Berichte 92, 1599 (1959).

Die folgenden Beispiele dienen zur Illustration der Erfindung, schränken deren Umfang jedoch in keiner Weise ein.

Beispiel 1: Reduktion von α-Benzoylaminomethyl-acetessigsäureethylester mit Bäckerhefe

In einem 500 ml Erlenmeyerkolben werden 200 ml Phosphatpuffer (pH 7,0), 30 g Bäckerhefe (Firma Klipfel) und 40 g Glucose bei 33 °C und 250 u.p.m. geschüttelt. Nach 30 Min. werden 20,7 g α-Benzoylaminomethyl-acetessigsäure-ethylester, nach 24 Stunden weitere 25 g Glucose und nach 48 Stunden nochmals 15 g Glucose zugegeben. Das Gemisch wird insgesamt 144 Stunden bei 33 °C geschüttelt. Die Reaktionslösung wird über Celite™ filtriert, die wässrige Lösung siebenmal mit Essigester extrahiert, die organische Phase über Natriumsulfat getrocknet und das Lösungsmittel am Rotationsverdampfer abgezogen. Der Rückstand wird durch Chromatographie an Kieselgel mit Toluol und Essigester aufgetrennt:

(2S,3S)-2-Benzoylaminomethyl-3-hydroxybuttersäure-ethylester;

Smp. 72-73 °C; $[\alpha]_D$ = -30,2 ° (Dioxan, c = 0,14%); UV (Ethanol)$\lambda_{max}$ = 226 nm (ε = 11540); IR ($CH_2Cl_2$) 3449, 1721, 1663, 1579 $cm^{-1}$; DC (Silicagel, Toluol/Essigester 1:2) $R_f$ = 0,32.

(2R,3S)-2-Benzoylaminomethyl-3-hydroxybuttersäure-ethylester;

Oel; $[\alpha]_D$ = -9,1 ° (Dioxan c = 0,14%); UV (Ethanol)$\lambda_{max}$ = 226 nm (ε = 11000); IR ($CH_2Cl_2$) 3444, 1719, 1647, 1601, 1578 $cm^{-1}$; DC (Silicagel, Toluol/Essigester 1:2) $R_f$ = 0,38.

Die Enantiomerenreinheit der (2S,3S)- und (2R,3S)-Ester wird nach Derivatisierung mit dem Mosher-Reagens R(+)-α-Methoxy-α-trifluormethyl-phenylessigsäurechlorid mit Proton-Kernresonanzspektroskopie und HPLC (high performance liquid chromatography) überprüft und beträgt über 99 %.

Die wässrige Phase wird mit 4 N HCl auf pH 2 eingestellt und die Säuren mit sieben 250 ml-Portionen Essigester extrahiert. Die vereinigten organischen Phasen werden über Natriumsulfat getrocknet und eingedampft. Der Rücktand wird durch Umkehrphasen-Chromatographie an Opti $UPC_{12}$®-Kieselgel mit Wasser aufgetrennt:

(2S,3S)-2-Benzoylaminomethyl-3-hydroxybuttersäure;

Smp. 99-101 °C (aus Essigester); $[\alpha]_D$ = -21,2 ° (Dioxan, c = 0,55%); UV (Ethanol)$\lambda_{max}$ = 227 nm (ε = 11360); IR ($CH_2Cl_2$) 3312, 1735, 1577, 1490 $cm^{-1}$; DC ($UPC_{12}$, Wasser/Acetonitril 9:1) $R_f$ = 0,25.

(2R,3S)-2-Benzoylaminomethyl-3-hydroxybuttersäure;

isolierte als Cyclohexylammoniumsalz;

$[\alpha]_D$ = +10 ° (Ethanol, c = 0,72%); UV (Ethanol)$\lambda_{max}$ = 227 nm (ε = 11000); IR ($CH_2Cl_2$) 3381, 1657, 1625, 1518, 1486 $cm^{-1}$; DC ($UPC_{12}$, Wasser/Acetonitril 9:1) $R_f$ = 0,33.

Die Säuren werden wie folgt verestert: 620 mg eines Gemisches der Säuren werden in 12 ml Ethanol mit 250 mg p-Toluolsulfonsäure 28 Stunden auf 45 °C erwärmt. Die Reaktionslösung wird eingeengt, mit Essigester verdünnt, mit gesättigter Natriumhydrogencarbonat-Lösung gewaschen, getrocknet und eingedampft. Die gebildeten Ethylester sind mit den obengenannten Estern identisch, $R_f$ (Silicagel, Toluol/Essigester 1:2) = 0,32 und 0,38, Enantiomerenreinheit über 99%.

Beispiel 2: Reduktion von α-Benzoylaminomethyl-acetessigsäureethylester mit Wasserstoff, Platin und einem chiralen Amin

40 mg Platinoxid und 0,28 ml R(+)-1-Phenylethylamin werden in 20 ml Toluol bei Normaldruck während 1 Stunde vorhydriert. Dann werden 4 g α-Benzoylaminomethyl-acetessigsäure-ethylester in 20 ml Toluol zugefügt und das Gemisch bei Normaldruck und Raumtemperatur während 22 Stunden hydriert. Die Reaktionslösung wird filtriert und eingedampft. Die Isomerenzusammensetzung des gebildeten 2-Benzoylaminomethyl-3-hydroxy-buttersäure-ethylesters wird durch Gaschromatographie an einer chiralen stationären Phase (Chirasil-Val®) bestimmt und beträgt (2S,3R):(2R,3S):(2S,3S):(2R,3R) = 13:14:24:49.

Wird anstelle von R(+)-1-Phenylethylamin das enantiomere S(-)-1-Phenylethylamin eingesetzt, so erhält

man ein Isomerenverhältnis von (2S,3R):(2R,3S):(2S,3S/2R,3R) = 10:21:69.

Beispiel 3: Reduktion von α-Acetylaminomethyl-acetessigsäureethylester mit Waserstoff, Platin und einem chiralen Amin

Analog Beispiel 2 werden 4,1 g α-Acetylaminomethyl-acetessigsäureethylester in Toluol über 41 mg Platinoxid in Gegenwart von 0,38 ml S(-)-1-Phenylethylamin hydriert und aufgearbeitet. Die gaschromatographische Analyse an Chirasil-Val® ergibt ein Isomerenverhältnis von (2S,3R):(2R,3S):(2S,3S):(2R,3R) = 20:19:22:39.
Mit R(+)-1-Phenylethylamin ergibt sich ein Isomerenverhältnis von (2S,3R):(2R,3S):(2S,3S):(2R,3R) = 16:22:22:39.

Beispiel 4: (5S,6R)-6-Methyl-2-phenyl-5,6-dihydro-1,3-4H-oxazinyl-5-carbonsäure-ethylester

Eine Lösung von 300 mg (2S,3S)-2-Benzoylaminomethyl-3-hydroxybuttersäure-ethylester in 0,6 ml Methylenchlorid wird mit 0,123 ml Thionylchlorid versetzt und bei Raumtemperatur während 3 Stunden gerührt. Die Reaktionslösung wird am Rotationsverdampfer eingeengt, in Toluol aufgenommen und eingedampft. Der Rückstand wird in wenig Essigester suspendiert und das kristallin anfallende Hydrochlorid der Titelverbindung, Smp. 125-126 ° C, abfiltriert.
$[\alpha]_D$ = +25,6 ° (Wasser, c = 0,09%), UV (Ethanol)$\lambda_{max}$ = 236 nm ($\epsilon$-11000); IR ($CH_2Cl_2$) 1737, 1656, 1379 $cm^{-1}$.

Das Hydrochlorid wird im Essigester aufgenommen und mit gesättigter Natriumbicarbonatlösung gewaschen. Die organische Phase wird mit Natrium getrocknet und die freie Base als Oel isoliert.
DC (Silicagel, Toluol/Essigester 1:1) $R_f$ = 0,69;
$[\alpha]_D$ = +81,4 ° (Dioxan, c = 0,30%); UV (Ethanol)$\lambda_{max}$ = 235 nm ($\epsilon$-13930); IR ($CH_2Cl_2$) 1727, 1659 $cm^{-1}$.

Beispiel 5: (5R,6R)-6-Methyl-2-phenyl-5,6-dihydro-1,3,4H-oxazinyl-5-carbonsäure-ethylester

Analog Biespiel 4 werden 1,6 g (2R,3S)-2-Benzoylaminomethyl-3-hydroxy-buttersäure-ethylester mit 0,66 ml Thionylchlorid in Methylenchlorid cyclisiert. Die Titelverbindung wird als Oel isoliert.
DC (Silicagel, Toluol/Essigester 1:1) $R_f$ = 0,55;
$[\alpha]_D$ = -9,6 ° (Dioxan, c = 0,31%); UV (Ethanol)λmax = 234 nm ($\epsilon$ = 12120); IR ($CH_2Cl_2$) 1730, 1658 $cm^{-1}$.

Beispiel 6: Isomerisierung der 6-Methyl-2-phenyl-5,6-dihydro-1,3,4H-oxazinyl-5-carbonsäure-ethylester

247 mg des (5R,6R)-Isomeren der Titelverbindung werden in 1 ml Dioxan gelöst und mit 0,05 ml 1,8-Diazabicyclo[5.4.0]undec-7-en versetzt. Das Gemisch wird während 7 Stunden auf 90 ° C erwärmt, anschliessend mit Toluol verdünnt und zweimal mit verdünnter Natriumchloridlösung gewaschen. Die organische Phase wird mit Natriumsulfat getrocknet, eingeengt, der Rückstand in Essigester aufgenommen und die Lösung mit HCl-Gas versetzt. Die ausfallenden Kristalle des Hydrochlorids des (5S,6R)-Isomeren der Titelverbindung werden abfiltriert und mit Essigester gewaschen, Smp. 125-126 ° C, identisch mit dem Produkt von Beispiel 4.
Das gleiche Produkt bei der Isomerisierung in Toluol (16 Stunden, 110 ° C) erhalten.

Das rohe Gemisch der (2S,3S)- und (2R,3S)-Isomeren von 2-Benzoylaminomethyl-3-hydroxy-buttersäure-ethylester aus der Bäckerhefe-Reduktion von Beispiel 1 kann direkt analog Beispiel 4 mit Thionylchlorid cyclisiert werden und mit 1,8-Diazabicyclo[5.4.0]undec-7-en in Toluol bei 110 ° C während 16 Stunden isomerisiert werden. Durch Einleiten von HCl-Gas in die Essigester-Lösung wird das Hydrochlorid des (5S,6R)-Isomeren der Titelverbindung, Smp. 125-126 ° C, erhalten.

Beispiel 7: (2S,3R)-2-Aminomethyl-3-hydroxy-buttersäure

3 g (5S,6R)-6-Methyl-2-phenyl-5,6-dihydro-1,3,4H-oxazinyl-5-carbonsäure-ethylester werden in 3 ml Wasser gelöst, mit 2 ml 16%iger Salzsäure versetzt und 5 Stunden am Rückfluss erhitzt. Die Lösung wird auf 4 ° C abgekühlt, die kristalline Benzoesäure abfiltriert, mit Wasser gewaschen und das Filtrat vollständig eingedampft. Der ölige Rückstand wird mit 4 Portionen von 20 ml Essigester ausgerührt, wobei das Hydrochlorid der Titelverbindung in eine kristalline Form übergeht und abfiltriert werden kann.
Smp. 120-123 ° C; DC ($UPC_{12}$, Wasser) $R_f$ = 0,8; $[\alpha]_D$ = -11,0 ° (Wasser, c = 0,81%); IR (KBr) 1719, 1623, 1486 $cm^{-1}$.

Beispiel 8: (3S,1′R)-3-(1′-Hydroxethyl)-2-azetidinon

Eine Suspension von 266 mg (2S,3R)-2-Aminomethyl-3-hydroxybuttersäure, 600 mg Triphenylphosphin, 50 mg 2-Mercaptopyridin und 200 mg Mangandioxid in 5 ml Acetonitril wird während 6 Stunden auf 70 °C erwärmt. Das Reaktionsgemisch wird eingeengt und mit Essigester an Kieselgel chromatographiert. Die Titelverbindung wird aus Tetrahydrofuran umkristallisiert, Smp. 101 °C; DC (Silicagel, Essigester) $R_f$ = 0,15; $[\alpha]_D$ = -64,2° (DMSO, c = 1,18%); IR ($CH_2Cl_2$)3604, 3415, 1758 $cm^{-1}$.

In einem grösseren Ansatz werden 15 g (2S,3R)-2-Aminomethyl-3-hydroxy-buttersäure-hydrochlorid in 50 ml Methylenchlorid aufgeschlämmt, mit 12,5 ml Triethylamin versetzt und 16 Stunden bei Raumtemperatur gerührt. Die freigesetzte Aminosäure wird abfiltriert und getrocknet. 1,26 g dieser Aminosäure werden in 10 ml Acetonitril mit 2,36 g Triphenylphosphin und 1,99 g 2,2′-Dipyridyldisulfid versetzt und 2 Stunden auf 80 °C erwärmt. Das Reaktionsgemisch wird am Rotationsverdampfer eingedampft und mit Essigester an 120 g Kieselgel chromatographiert. Smp. 101 °C (aus Tetrahydrofuran).

Beispiel 9: (3R,4R,1′R)-4-Acetoxy-3-(1′-hydroxyethyl)-2-azetidinon

Eine Lösung von 4 g (3S,1′R)-3-(1′-Hydroxyethyl)-2-azetidinon und 1 g Tetrabutylammoniumtetrafluoroborat in 50 ml Eisessig und 1 ml Wasser wird während 4 Stunden an Platinelektroden in einer ungeteilten Zelle bei einer Stromdichte von 50 mA/$cm^2$ elektrolysiert. Das Lösungsmittel wird mit Toluol am Rotationsverdampfer entfernt und der Rückstand durch Chromatographie an Kieselgel mit Essigester gereinigt. Smp. 103-105 °C; $[\alpha]_D$ = +59,7° (Chloroform, c = 0,36%); UV (Chloroform)$\lambda_{max}$ = 269 nm ($\epsilon$=73); IR (Chloroform) 3602, 3406, 1784, 1366 $cm^{-1}$; DC (Silicagel, Essigester) $R_f$ = 0,51.

Die Elektrolyse kann statt in Eisessig auch in folgenden Lösungsmittelgemischen durchgeführt werden: Eisessig/Acetonitril 1:1 und 1:9, Eisessig/Acetonitril/Essigester 1:2:9. Als Leitsalze eignen sich ebenfalls Natriumtetrafluoroborat, Lithiumtetrafluoroborat und Ammoniumnitrat. Als Anode können Platin (Folie, Netz, Draht), VA4 und Glaskohlenstoff eingesetzt werden.

Beispiel 10: 4(R)-(N-Allyloxycarbonylglycylthio)-3-(S)-(1′(R)-hydroxyethyl)-2-azetidinon

Eine Lösung von 426 mg N-Allyloxycarbonyl-thioglycin-dicyclohexylammoniumsalz in 1,2 ml 1 N wässriger Natronlauge wird dreimal mit 1,5 ml $CH_2Cl_2$ gewaschen und mit 0,1 N Salzsäure auf pH 8-9 gestellt. Die erhaltene wässrige Thiolsäurelösung wird bei 25 °C zu einer Lösung von 173,2 mg (3R,4R,1′R)-4-Acetoxy-3-(1′hydroxethyl)-2-azetidinon in 1,7 ml Acetonitril zufliessen gelassen. Nach Zugabe von 0,1 ml 0,1 N wässriger Natronlauge wird 35 Minuten bei 21-23 °C weitergerührt. Zur Aufarbeitung werden in einem Scheidetrichter 250 ml Essigsäureethylester und 30 g NaCl vorgelegt und dazu das Reaktionsgemisch gegeben. Nach gutem Schütteln und Abtrennen der wässrigen Phase wird die organische Phase noch einmal mit 50 ml 5%iger wässriger $NaHCO_3$-Lösung und zweimal mit 50 ml Sole gewaschen und über Natriumsulfate getrocknet. Das Lösungsmittel wird am Rotationsverdampfer entfernt. Man erhält die Titelverbindung als amorphes Pulver. Das Rohprodukt kann an Kieselgel chromatographisch gereinigt werden (Toluol/Essigester 2:3). $R_f$-Wert 0,23 (Merck Fertigplatten, Toluol/Essigester = 1:4 Ninhydrin als Entwicklungsreagens).

**Patentansprüche**

**Patentansprüche für folgende Vertragsstaaten : BE, CH, DE, FR, GB, IT, LI, NL, SE**

**1.** Verfahren zur Herstellung von Verbindungen der Formel

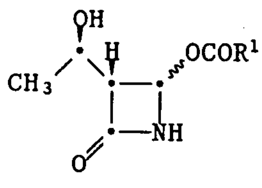

(I),

worin $R^1$ $C_1$-$C_7$-Niederalkyl oder Aryl, das 6 bis 14 C-Atome enthält und durch $C_1$-$C_7$-Niederalkyl, Hydroxy, $C_1$-$C_7$-Niederalkoxy, $C_1$-$C_8$-Niederalkanoyloxy, Halogen oder Nitro substituiert sein kann, bedeutet, dadurch gekennzeichnet, dass man eine Verbindung der Formel

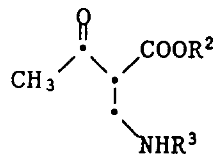

(II),

worin $R^2$ Wasserstoff oder einen veresternden Rest und $R^3$ Wasserstoff oder einen Acylrest aus der Gruppe $C_1$-$C_8$-Niederalkanoyl, $C_3$-$C_8$-Cycloalkylcarbonyl, $C_6$-$C_{14}$-Arylcarbonyl, $C_1$-$C_7$-Niederalkoxycarbonyl, $C_6$-$C_{14}$-Aryloxycarbonyl und $C_6$-$C_{14}$-Aryl-$C_1$-$C_7$-niederalkoxycarbonyl bedeuten, oder ein Salz davon mit einem enantioselektiven Reduktionsmittel reduziert, eine erhältliche Verbindung der Formel

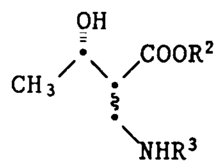

(IIIa),

falls $R^2$ und/oder $R^3$ Wasserstoff bedeuten, in beliebiger Reihenfolge verestert beziehungsweise mit einem den Acylrest $R^3$ einführenden Reagens acyliert, und die Verbindung der Formel IIIa, worin $R^2$ einen veresternden Rest und $R^3$ einen Acylrest $R^3$ bedeuten, oder ein Salz davon mit einem die Hydroxygruppe aktivierenden Mittel cyclisiert, eine erhältliche Verbindung der Formel

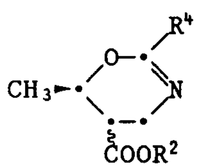

(IV),

worin $R^2$ einen veresternden Rest und $R^4$ $C_1$-$C_7$-Niederalkyl, $C_3$-$C_8$-Cycloalkyl, $C_6$-$C_{14}$-Aryl, $C_1$-$C_7$-Niederalkoxy, $C_6$-$C_{14}$-Aryloxy oder $C_6$-$C_{14}$-Arylniederalkoxy, worin Aryl wie unter $R^1$ angegeben substituiert sein kann, bedeuten, mit Base isomerisiert und ein erhältliches Diastereomeres der Formel

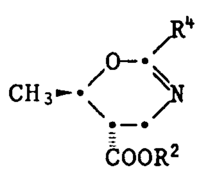

(IVa)

hydrolysiert, die erhältliche Verbindung der Formel

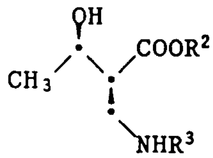

(IIIb),

worin $R^2$ und $R^3$ Wasserstoff bedeuten, oder ein Salz davon mit einem wasserentziehenden Mittel cyclisiert, und die erhältliche Verbindung der Formel

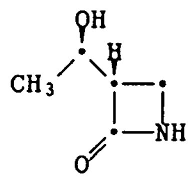

(V)

mit einem den Rest $R^1COO$- einführenden Mittel oxidiert.

2. Verfahren gemäss Anspruch 1 zur Herstellung von (3R,4R,1'R)-4-Acetoxy-3-(1'-hydroxyethyl)-2-azetidinon der Formel

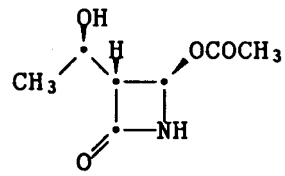

(Ia).

3. Verfahren gemäss Anspruch 1 oder 2, dadurch gekennzeichnet, dass ein veresternder Rest $R^2$ $C_1$-$C_4$-Niederalkyl, der Acylrest $R^3$ $C_2$-$C_5$-Niederalkanoyl oder $C_6$-$C_{10}$-Arylcarbonyl, das durch Methyl, Methoxy oder Dinitro substituiert sein kann, und $R^4$ $C_1$-$C_4$-Niederalkyl beziehungsweise $C_6$-$C_{10}$-Aryl, das durch Methyl, Hydroxy, Methoxy, Acetoxy, Chlor, Brom oder Nitro substituiert sein kann, bedeuten.

4. Verfahren zur Herstellung von Verbindungen der Formel

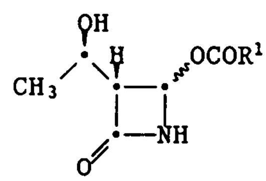

(I),

worin $R^1$ die im Anspruch 1 angegebene Bedeutung hat, dadurch gekennzeichnet, dass man die Verbindung der Formel

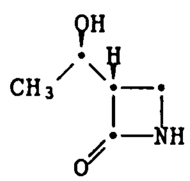

(V)

elektrochemisch in Gegenwart einer Säure $R^1COOH$ bei Stromdichten zwischen 10 und 400 mA/cm$^2$ und Temperaturen zwischen 0° und 50°C oxidativ acyliert.

5. Verfahren gemäss Anspruch 4 zur Herstellung von (3R,4R,1'R)-4-Acetoxy-3-(1'-hydroxyethyl)-2-azetidinon der Formel

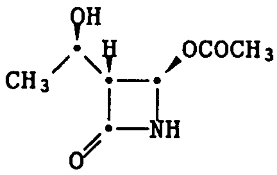

(Ia).

6. Verfahren zur Herstellung von Verbindungen der Formel

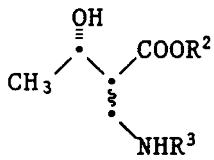

(IIIa),

worin $R^2$ und $R^3$ die im Anspruch 1 angegebenen Bedeutungen haben, dadurch gekennzeichnet, dass man eine Verbindung der Formel

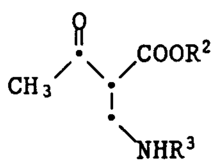

(II),

worin $R^2$ und $R^3$ die genannten Bedeutungen haben, oder ein Salz davon mit Bäckerhefe reduziert, und gewünschtenfalls eine erhältliche Verbindung der Formel IIIa in eine andere erfindungsgemässe Verbindung der Formel IIIa umwandelt, und gegebenenfalls eine erhältliche freie Verbindung in ein Salz oder ein erhältliches Salz in eine freie Verbindung oder in ein anderes Salz umwandelt.

7. Verfahren gemäss Anspruch 6, dadurch gekennzeichnet, dass ein veresternder Rest $R^2$ und der Acylrest $R^3$ die im Anspruch 3 angegebenen Bedeutungen haben.

8. Verbindungen der Formel

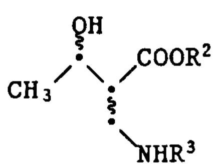

(III),

worin $R^2$ und $R^3$ die im Anspruch 1 angegebenen Bedeutungen haben, deren reine Diastereomere, Gemische zweier Diastereomere, und Salze davon.

9. Verbindungen gemäss Anspruch 8 der Formel III, worin $R^2$ und $R^3$ Wasserstoff oder die im Anspruch 3 angegebenen Bedeutungen haben, deren reine Diastereomere, Gemische zweier Diastereomere, und Salze davon.

10. Verbindungen gemäss Anspruch 8 der Formel

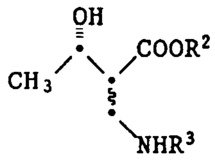

(IIIa),

worin $R^2$ Wasserstoff oder $C_1$-$C_7$-Niederalkyl und $R^3$ $C_1$-$C_8$-Niederalkanoyl oder $C_6$-$C_{14}$-Arylcarbonyl, das durch Methyl, Methoxy oder Dinitro substituiert sein kann, bedeuten, deren reine Diastereomere, und Salze davon.

**11.** Die Verbindung gemäss Anspruch 8 der Formel

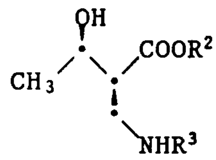

(IIIb),

worin $R^2$ und $R^3$ Wasserstoff bedeuten, und deren Salze.

**12.** Verbindungen der Formel

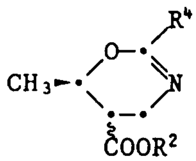

(IV),

worin $R^2$ und $R^4$ die im Anspruch 1 angegebenen Bedeutungen haben, deren reine Diastereomere und Salze davon.

**13.** Verbindungen gemäss Anspruch 12 der Formel IV, worin $R^2$ $C_1$-$C_4$-Niederalkyl und $R^4$ $C_1$-$C_4$-Niederalkyl oder $C_6$-$C_{10}$-Aryl, das durch Methyl, Hydroxy, Methoxy, Acetoxy, Chlor, Brom oder Nitro substituiert sein kann, bedeuten, deren reine Diastereomere, und Salze davon.

**14.** Die Verbindung der Formel

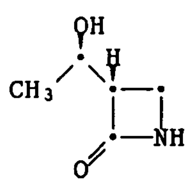

(V).

## Patentansprüche für folgenden Vertragsstaat : ES

**1.** Verfahren zur Herstellung von Verbindungen der Formel

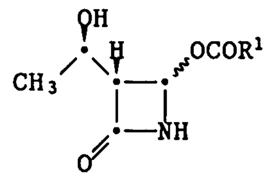

(I),

worin $R^1$ $C_1$-$C_7$-Niederalkyl oder Aryl das 6 bis 14 C-Atome enthält und durch $C_1$-$C_7$-Niederalkyl, Hydroxy, $C_1$-$C_7$-Niederalkoxy, $C_1$-$C_8$-Niederalkanoyloxy, Halogen oder Nitro substituiert sein kann, bedeutet, dadurch gekennzeichnet, dass man eine Verbindung der Formel

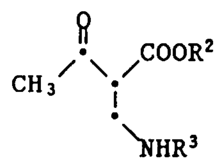

(II),

worin $R^2$ Wasserstoff oder einen veresternden Rest und $R^3$ Wasserstoff oder einen Acylrest aus der Gruppe $C_1$-$C_8$-Niederalkanoyl, $C_3$-$C_8$-Cycloalkylcarbonyl, $C_6$-$C_{14}$-Arylcarbonyl, $C_1$-$C_7$-Niederalkoxycarbonyl, $C_6$-$C_{14}$-Aryloxycarbonyl und $C_6$-$C_{14}$-Aryl-$C_1$-$C_7$-niederalkoxycarbonyl bedeuten, oder ein Salz davon mit einem enantioselektiven Reduktionsmittel reduziert, eine erhältliche Verbindung der Formel

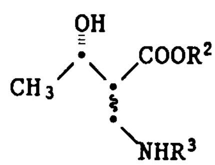

(IIIa),

falls $R^2$ und/oder $R^3$ Wasserstoff bedeuten, in beliebiger Reihenfolge verestert beziehungsweise mit einem den Acylrest $R^3$ einführenden Reagens acyliert, und die Verbindung der Formel IIIa, worin $R^2$ einen veresternden Rest und $R^3$ einen Acylrest $R^3$ bedeuten, oder ein Salz davon mit einem die Hydroxygruppe aktivierenden Mittel cyclisiert, eine erhältliche Verbindung der Formel

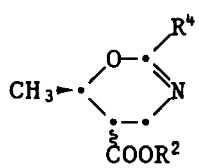

(IV),

worin $R^2$ einen veresternden Rest und $R^4$ $C_1$-$C_7$-Niederalkyl, $C_3$-$C_8$-Cycloalkyl, $C_6$-$C_{14}$-Aryl, $C_1$-$C_7$-Niederalkoxy, $C_6$-$C_{14}$-Aryloxy oder $C_6$-$C_{14}$-Arylniederalkoxy, worin Aryl wie unter $R^1$ angegeben substituiert sein kann, bedeuten, mit Base isomerisiert und ein erhältliches Diastereomeres der Formel

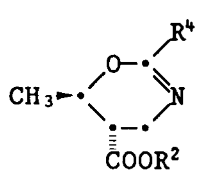

(IVa)

hydrolysiert, die erhältliche Verbindung der Formel

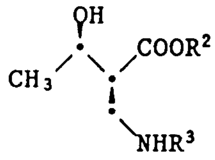

(IIIb),

worin $R^2$ und $R^3$ Wasserstoff bedeuten, oder ein Salz davon mit einem wasserentziehenden Mittel cyclisiert, und die erhältliche Verbindung der Formel

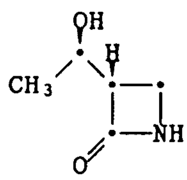

(V)

mit einem den Rest $R^1COO$- einführenden Mittel oxidiert.

**2.** Verfahren gemäss Anspruch 1 zur Herstellung von (3R,4R,1'R)-4-Acetoxy-3-(1'-hydroxyethyl)-2-azetidinon der Formel

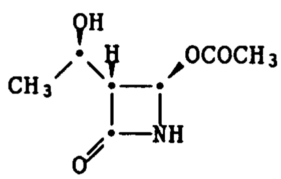

(Ia).

**3.** Verfahren gemäss Anspruch 1 oder 2, dadurch gekennzeichnet, dass ein veresternder Rest $R^2$ $C_1$-$C_4$-Niederalkyl, der Acylrest $R^3$ $C_2$-$C_5$-Niederalkanoyl oder $C_6$-$C_{10}$-Arylcarbonyl, das durch Methyl, Methoxy oder Dinitro substituiert sein kann, und $R^4$ $C_1$-$C_4$-Niederalkyl beziehungsweise $C_6$-$C_{10}$-Aryl, das durch Methyl, Hydroxy, Methoxy, Acetoxy, Chlor, Brom oder Nitro substituiert sein kann, bedeuten.

**4.** Verfahren zur Herstellung von Verbindungen der Formel

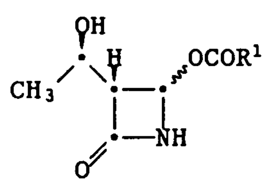

(I),

worin $R^1$ die im Anspruch 1 angegebene Bedeutung hat, dadurch gekennzeichnet, dass man die Verbindung der Formel

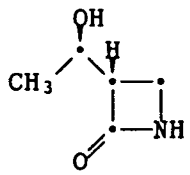

(V)

elektrochemisch in Gegenwart einer Säure $R^1COOH$ bei Stromdichten zwischen 10 und 400 mA/cm$^2$ und Temperaturen zwischen 0° und 50° C oxidativ acyliert.

5. Verfahren gemäss Anspruch 4 zur Herstellung von (3R,4R,1'R)-4-Acetoxy-3-(1'-hydroxyethyl)-2-azetidinon der Formel

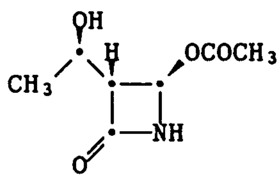

(Ia).

6. Verfahren zur Herstellung von Verbindungen der Formel

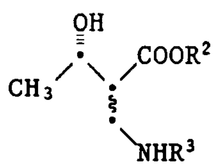

(IIIa),

worin $R^2$ und $R^3$ die im Anspruch 1 angegebenen Bedeutungen haben, dadurch gekennzeichnet, dass man eine Verbindung der Formel

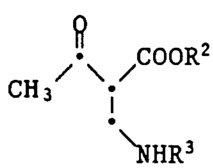

(II),

worin $R^2$ und $R^3$ die genannten Bedeutungen haben, oder ein Salz davon mit einem enantioselektiven Reduktionsmittel reduziert, und gewünschtenfalls eine erhältliche Verbindung der Formel IIIa in eine andere erfindungsgemässe Verbindung der Formel IIIa umwandelt, und gegebenenfalls eine erhältliche freie Verbindung in ein Salz oder ein erhältliches Salz in eine freie Verbindung oder in ein anderes Salz umwandelt.

7. Verfahren gemäss Anspruch 6, dadurch gekennzeichnet, dass als enantioselektives Reduktionsmittel Bäckerhefe verwendet wird.

8. Verfahren gemäss Anspruch 6 oder 7, dadurch gekennzeichnet, dass ein veresternder Rest $R^2$ und der Acylrest $R^3$ die im Anspruch 3 angegebenen Bedeutungen haben.

9. Verfahren zur Herstellung von Verbindungen der Formel

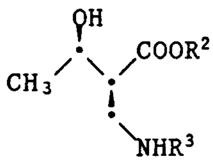

(IIIb),

worin $R^2$ und $R^3$ Wasserstoff bedeuten, und deren Salze, dadurch gekennzeichnet, dass man eine Verbindung der Formel

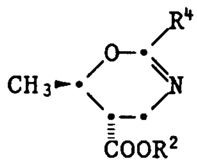

(IVa),

worin $R^2$ und $R^4$ die im Anspruch 1 angegebenen Bedeutungen haben, hydrolysiert, und gegebenenfalls eine erhältliche freie Verbindung in ein Salz oder ein erhältliches Salz in eine freie Verbindung oder in ein anderes Salz umwandelt.

**10.** Verfahren zur Herstellung von Verbindungen der Formel

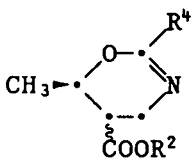

(IV),

worin $R^2$ und $R^4$ die im Anspruch 1 angegebenen Bedeutungen haben, deren reinen Diastereomere und Salzen davon, dadurch gekennzeichnet, dass man eine Verbindung der Formel

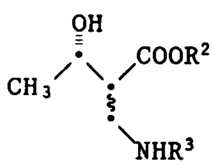

(IIIa),

worin $R^2$ und $R^3$ die im Anspruch 1 angegebenen Bedeutungen haben, oder ein Salz davon mit einem die Hydroxygruppen aktivierenden Mittel cyclisiert und gewünschtenfalls ein erhältliches Diastereomeres oder Diastereomerengemisch mit Base isomerisiert, und gegebenenfalls eine erhältliche freie Verbindung in ein Salz oder ein erhältliches Salz in eine freie Verbindung oder in ein anderes Salz umwandelt

**11.** Verfahren gemäss Anspruch 10, dadurch gekennzeichnet, dass $R^2$ $C_1$-$C_4$-Niederalkyl, $R^3$ $C_1$-$C_4$-Niederalkanoyl oder $C_6$-$C_{10}$-Arylcarbonyl, das durch Methyl, Methoxy oder Dinitro substituiert sein kann, und $R^4$ $C_1$-$C_4$-Niederalkyl oder Aryl, das durch Methyl, Hydroxy, Methoxy, Acetoxy, Chlor, Brom oder Nitro substituiert sein kann, bedeuten.

**12.** Verfahren zur Herstellung der Verbindung der Formel

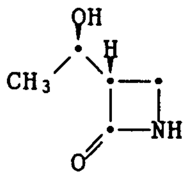

(V).

dadurch gekennzeichnet, dass man die Verbindung der Formel

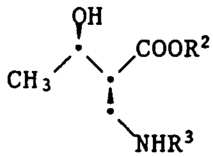

(IIIb),

worin $R^2$ und $R^3$ Wasserstoff bedeuten, oder ein Salz davon mit einem wasserentziehenden Mittel cyclisiert.

**Claims**

**Claims for the following Contracting States : BE, CH, DE, FR, GB, IT, LI, NL, SE**

**1.** A process for the manufacture of a compound of formula

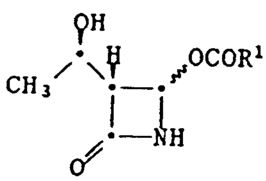

(I),

in which $R^1$ represents $C_1$-$C_7$lower alkyl or aryl that contains from 6 to 14 carbon atoms and can be substituted by $C_1$-$C_7$lower alkyl, hydroxy, $C_1$-$C_7$lower alkoxy, $C_1$-$C_8$lower alkanoyloxy, halogen or by nitro, characterised in that a compound of formula

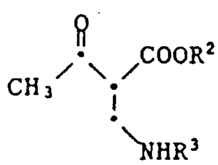

(II),

in which $R^2$ represents hydrogen or an esterifying radical and $R^3$ represents hydrogen or an acyl radical selected from the group $C_1$-$C_8$lower alkanoyl, $C_3$-$C_8$cycloalkylcarbonyl, $C_6$-$C_{14}$arylcarbonyl, $C_1$-$C_7$lower alkoxycarbonyl, $C_6$-$C_{14}$aryloxycarbonyl and $C_6$-$C_{14}$aryl-$C_1$-$C_7$lower alkoxycarbonyl, or a salt thereof, is reduced with an enantioselective reducing agents an obtainable compound of formula

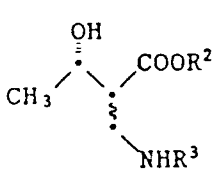

(IIIa),

in the case when $R^2$ and/or $R^3$ represent(s) hydrogen, is in any sequence esterified or acylated with a reagent that introduces the acyl radical $R^3$, and the compound of formula IIIa in which $R^2$ represents an esterifying radical and $R^3$ represents an acyl radical $R^3$, or a salt thereof, is cyclised with an agent that activates the hydroxy group, an obtainable compound of formula

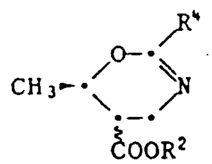

(IV),

in which $R^2$ represents an esterifying radical and $R^4$ represents $C_1$-$C_7$lower alkyl, $C_3$-$C_8$cycloalkyl, $C_6$-$C_{14}$aryl, $C_1$-$C_7$lower alkoxy, $C_6$-$C_{14}$aryloxy or $C_6$-$C_{14}$aryl-lower alkoxy, wherein aryl can be substituted as indicated under $R^1$, is isomerised with a base, and an obtainable diastereoisomer of formula

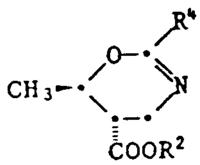

(IVa)

is hydrolysed, an obtainable compound of formula

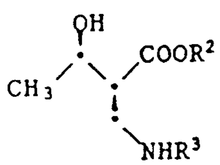

(IIIb),

in which $R^2$ and $R^3$ represent hydrogen, or a salt thereof, is cyclised with an agent removing the elements of water, and an obtainable compound of formula

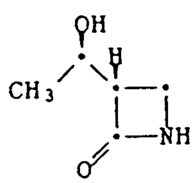

(V)

is oxidised with an agent that introduces the radical $R^1COO$-.

2. A process according to claim 1 for the manufacture of (3R,4R,1'R)-4-acetoxy-3-(1'-hydroxyethyl)-2-azetidinone of formula

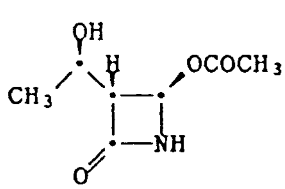

(Ia).

3. A process according to claim 1 or 2, characterised in that an esterifying radical $R^2$ is $C_1$-$C_4$lower alkyl, the acyl radical $R^3$ is $C_2$-$C_5$lower alkanoyl or $C_6$-$C_{10}$arylcarbonyl that can be substituted by methyl,

methoxy or by dinitro, and $R^4$ is $C_1$-$C_4$lower alkyl or $C_6$-$C_{10}$aryl that can be substituted by methyl, hydroxy, methoxy, acetoxy, chlorine, bromine or by nitro.

**4.** A process for the manufacture of a compound of formula

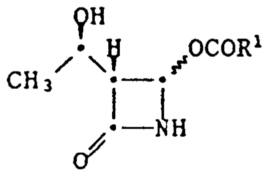

(I),

in which $R^1$ is as defined in claim 1, characterised in that the compound of formula

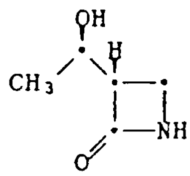

(V)

is oxidatively acylated electrochemically in the presence of an acid $R^1COOH$ at current densities of from 10 to 400 mA/cm$^2$ and temperatures of from 0° to 50°C.

**5.** A process according to claim 4 for the manufacture of (3R,4R,1'R)-4-acetoxy-3-(1'-hydroxyethyl)-2-azetidinone of formula

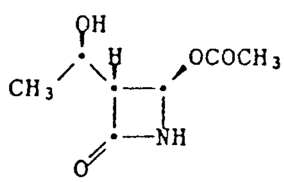

(Ia).

**6.** A process for the manufacture of a compound of formula

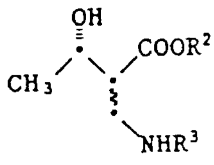

(IIIa),

in which $R^2$ and $R^3$ are as defined in claim 1, characterised in that a compound of formula

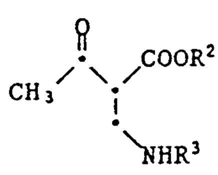

(II),

in which $R^2$ and $R^3$ are as defined, or a salt thereof, is reduced with baker's yeast and, if desired, an obtainable compound of formula IIIa is converted into a different compound of formula IIIa according to

the invention and, if desired, an obtainable free compound is converted into a salt or an obtainable salt is converted into a free compound or into a different salt.

**7.** A process according to claim 6, characterised in that an esterifying radical $R^2$ and the acyl radical $R^3$ are as defined in claim 3.

**8.** A compound of formula

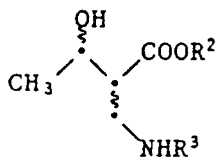

(III),

in which $R^2$ and $R^3$ are as defined in claim 1, a pure diastereoisomer, a mixture of two diastereoisomers or a salt thereof.

**9.** A compound according to claim 8 of formula III, in which $R^2$ and $R^3$ are hydrogen or are as defined in claim 3, a pure diastereoisomer, a mixture of two diastereoisomers or a salt thereof.

**10.** A compound according to claim 8 of formula

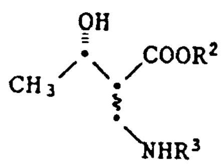

(IIIa),

in which $R^2$ represents hydrogen or $C_1$-$C_7$lower alkyl and $R^3$ represents $C_1$-$C_8$lower alkanoyl or $C_6$-$C_{14}$arylcarbonyl that can be substituted by methyl, methoxy or by dinitro, a pure diastereoisomer or a salt thereof.

**11.** The compound according to claim 8 of formula

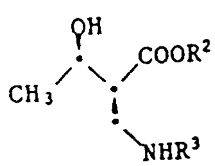

(IIIb),

in which $R^2$ and $R^3$ represent hydrogen, or a salt thereof.

**12.** A compound of formula

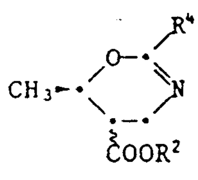

(IV),

in which $R^2$ and $R^4$ are as defined in claim 1, a pure diastereoisomer or a salt thereof.

**13.** A compound according to claim 12 of formula IV, in which $R^2$ represents $C_1$-$C_4$lower alkyl and $R^4$

represents $C_1$-$C_4$ lower alkyl or $C_6$-$C_{10}$ aryl that can be substituted by methyl, hydroxy, methoxy, acetoxy, chlorine, bromine or by nitro, a pure diastereoisomer or a salt thereof.

**14.** The compound of formula

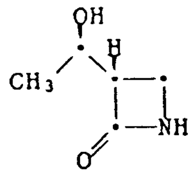

(V).

**Claims for the following Contracting State : ES**

**1.** A process for the manufacture of a compound of formula

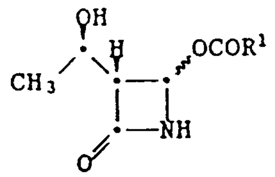

(I),

in which $R^1$ represents $C_1$-$C_7$ lower alkyl or aryl that contains from 6 to 14 carbon atoms and can be substituted by $C_1$-$C_7$ lower alkyl, hydroxy, $C_1$-$C_7$ lower alkoxy, $C_1$-$C_8$ lower alkanoyloxy, halogen or by nitro, characterised in that a compound of formula

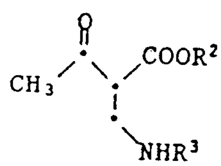

(II),

in which $R^2$ represents hydrogen or an esterifying radical and $R^3$ represents hydrogen or an acyl radical selected from the group $C_1$-$C_8$ lower alkanoyl, $C_3$-$C_8$ cycloalkylcarbonyl, $C_6$-$C_{14}$ arylcarbonyl, $C_1$-$C_7$ lower alkoxycarbonyl, $C_6$-$C_{14}$ aryloxycarbonyl and $C_6$-$C_{14}$ aryl-$C_1$-$C_7$ lower alkoxycarbonyl, or a salt thereof, is reduced with an enantioselective reducing agent, an obtainable compound of formula

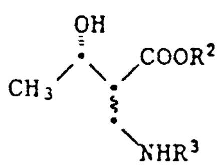

(IIIa),

in the case when $R^2$ and/or $R^3$ represent(s) hydrogen, is in any sequence esterified or acylated with a reagent that introduces the acyl radical $R^3$, and the compound of formula IIIa in which $R^2$ represents an esterifying radical and $R^3$ represents an acyl radical $R^3$, or a salt thereof, is cyclised with an agent that activates the hydroxy group, an obtainable compound of formula

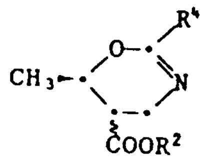

(IV),

in which $R^2$ represents an esterifying radical and $R^4$ represents $C_1$-$C_7$lower alkyl, $C_3$-$C_8$cycloalkyl, $C_6$-$C_{14}$aryl, $C_1$-$C_7$lower alkoxy, $C_6$-$C_{14}$aryloxy or $C_6$-$C_{14}$aryl-lower alkoxy, wherein aryl can be substituted as indicated under $R^1$, is isomerised with a base, and an obtainable diastereoisomer of formula

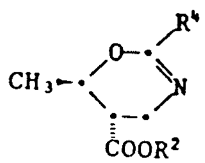

(IVa)

is hydrolysed, an obtainable compound of formula

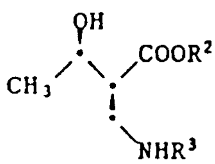

(IIIb),

in which $R^2$ and $R^3$ represent hydrogen, or a salt thereof, is cyclised with an agent removing the elements of water, and an obtainable compound of formula

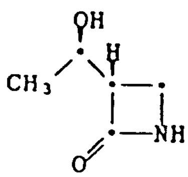

(V)

is oxidised with an agent that introduces the radical $R^1COO$-.

2. A process according to claim 1 for the manufacture of (3R,4R,1'R)-4-acetoxy-3-(1'-hydroxyethyl)-2-azetidinone of formula

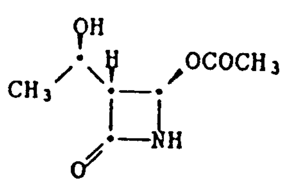

(Ia).

3. A process according to claim 1 or 2, characterised in that an esterifying radical $R^2$ is $C_1$-$C_4$lower alkyl, the acyl radical $R^3$ is $C_2$-$C_5$lower alkanoyl or $C_6$-$C_{10}$arylcarbonyl that can be substituted by methyl, methoxy or by dinitro, and $R^4$ is $C_1$-$C_4$lower alkyl or $C_6$-$C_{10}$aryl that can be substituted by methyl, hydroxy, methoxy, acetoxy, chlorine, bromine or by nitro.

4. A process for the manufacture of a compound of formula

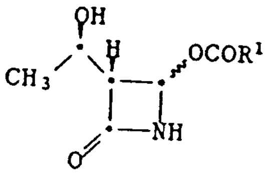

(I),

in which $R^1$ is as defined in claim 1, characterised in that the compound of formula

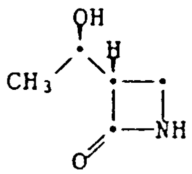

(V)

is oxidatively acylated electrochemically in the presence of an acid $R^1COOH$ at current densities of from 10 to 400 $mA/cm^2$ and temperatures of from 0° to 50°C.

**5.** A process according to claim 4 for the manufacture of (3R,4R,1'R)-4-acetoxy-3-(1'-hydroxyethyl)-2-azetidinone of formula

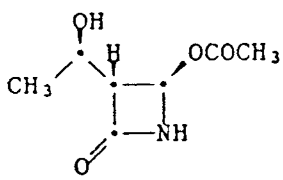

(Ia).

**6.** A process for the manufacture of a compound of formula

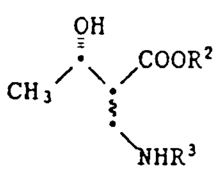

(IIIa),

in which $R^2$ and $R^3$ are as defined in claim 1, characterised in that a compound of formula

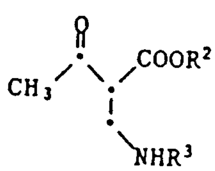

(II),

in which $R^2$ and $R^3$ are as defined, or a salt thereof, is reduced with an enantioselective reducing agent and, if desired, an obtainable compound of formula IIIa is converted into a different compound of formula IIIa according to the invention and, if desired, an obtainable free compound is converted into a salt or an obtainable salt is converted into a free compound or into a different salt.

**7.** A process according to claim 6, characterised in that the enantioselective reducing agent used is baker's yeast.

**8.** A process according to claim 6 or 7, characterised in that an esterifying radical $R^2$ and the acyl radical $R^3$ are as defined in claim 3.

**9.** A process for the manufacture of a compound of formula

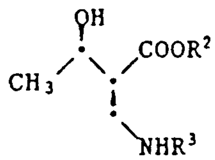

(IIIb),

in which $R^2$ and $R^3$ represent hydrogen, or a salt thereof, characterised in that a compound of formula

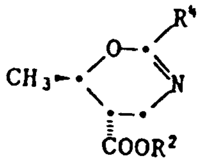

(IVa),

in which $R^2$ and $R^4$ are as defined in claim 1, is hydrolysed and, if desired, an obtainable free compound is converted into a salt or an obtainable salt is converted into a free compound or into a different salt.

**10.** A process for the manufacture of a compound of formula

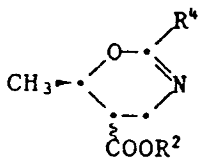

(IV),

in which $R^2$ and $R^4$ are as defined in claim 1, a pure diastereoisomer or a salt thereof, characterised in that a compound of formula

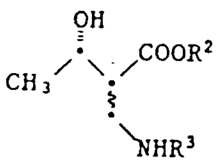

(IIIa),

in which $R^2$ and $R^3$ are as defined in claim 1, or a salt thereof, is cyclised with an agent that activates the hydroxy group and, if desired, an obtainable diastereoisomer or diastereoisomeric mixture is isomerised with a base and, if desired, an obtainable free compound is converted into a salt or an obtainable salt is converted into a free compound or into a different salt.

**11.** A process according to claim 10, characterised in that $R^2$ is $C_1$-$C_4$ lower alkyl, $R^3$ is $C_1$-$C_4$ lower alkanoyl or $C_6$-$C_{10}$ arylcarbonyl that can be substituted by methyl, methoxy or by dinitro, and $R^4$ is $C_1$-$C_4$ lower alkyl or aryl that can be substituted by methyl, hydroxy, methoxy, acetoxy, chlorine, bromine or by nitro.

**12.** A process for the manufacture of the compound of formula

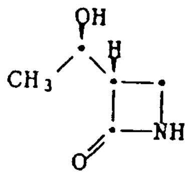

(V),

characterised in that a compound of formula

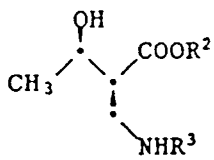

(IIIb),

in which $R^2$ and $R^3$ represent hydrogen, or a salt thereof, is cyclised with an agent removing the elements of water.

**Revendications**

**Revendications pour les Etats contractants suivants : BE, CH, DE, FR, GB, IT, LI, NL, SE**

**1.** Procédé de préparation des composés de formule :

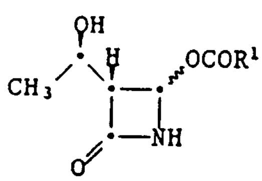

(I),

dans laquelle $R^1$ représente un groupe alkyle inférieur en $C_1$-$C_7$ ou aryle en $C_6$-$C_{14}$ éventuellement substitué par des groupes alkyle inférieurs en $C_1$-$C_7$, hydroxy, alcoxy inférieurs en $C_1$-$C_7$, alcanoyloxy inférieurs en $C_1$-$C_8$, des halogènes ou des groupes nitro, caractérisé en ce que l'on réduit un composé de formule :

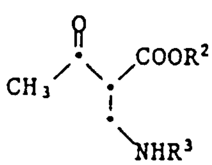

(II),

dans laquelle $R^2$ représente l'hydrogène ou un radical estérifiant et $R^3$ représente l'hydrogène ou un radical acyle choisi parmi les radicaux alcanoyle inférieurs en $C_1$-$C_8$, cycloalkylcarbonyle en $C_3$-$C_8$, arylcarbonyle en $C_6$ -$C_{14}$, (alcoxy inférieur en $C_1$-$C_7$)-carbonyle, aryloxycarbonyle en $C_6$-$C_{14}$ et (aryle en $C_6$-$C_{14}$) - (alcoxy inférieur en $C_1$-$C_7$)-carbonyle, ou un sel d'un tel composé, à l'aide d'un agent réducteur énantiosélectif, ce qui donne un composé de formule :

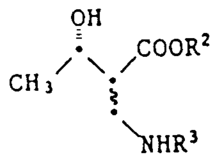

(IIIa),

qui, lorsque $R^2$ et/ou $R^3$ représentent l'hydrogène, est, dans un ordre quelconque, respectivement estérifié et acylé par un réactif introduisant le radical acyle $R^3$, et le composé de formule IIIa dans laquelle $R^2$ représente un radical estérifiant et $R^3$ un radical acyle $R^3$, ou un sel d'un tel composé, est cyclisé à l'aide d'un agent activant le groupe hydroxy, ce qui donne un composé de formule :

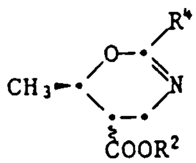

(IV),

dans laquelle $R^2$ représente un radical estérifiant et $R^4$ un groupe alkyle inférieur en $C_1$-$C_7$, cycloalkyle en $C_3$-$C_8$, aryle en $C_6$-$C_{14}$, alcoxy inférieur en $C_1$-$C_7$, aryloxy en $C_6$-$C_{14}$ ou (aryle en $C_6$-$C_{14}$) - alcoxy inférieur, les parties aryle pouvant être substituées comme indiqué en référence à $R^1$, on isomérise à l'aide d'une base, ce qui donne le diastéréoisomère de formule :

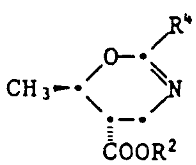

(IVa)

qu'on hydrolyse, ce qui donne le composé de formule :

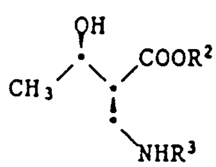

(IIIb),

dans laquelle $R^2$ et $R^3$ représentent l'hydrogène, ou un sel d'un tel composé, qu'on cyclise à l'aide d'un agent déshydratant, ce qui donne le composé de formule :

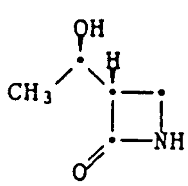

(V)

qu'on oxyde à l'aide d'un agent introduisant le radical $R^1COO$.

**2.** Procédé selon la revendication 1, pour la préparation de la (3R,4R,1'R)-4-acétoxy-3-(1'-hydroxyéthyl)-2azétidinone de formule :

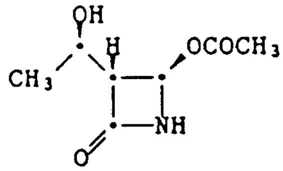

(Ia).

**3.** Procédé selon la revendication 1 ou 2, caractérisé en ce que le radical estérifiant $R^2$ est un radical alkyle inférieur en $C_1$-$C_4$, le radical acyle $R^3$ est un radical alcanoyle inférieur en $C_2$-$C_5$ ou arylcarbonyle en $C_6$-$C_{10}$, qui peut être substitué par des groupes méthyle, méthoxy ou dinitro, et $R^4$ représente respectivement un groupe alkyle inférieur en $C_1$-$C_4$ ou aryle en $C_6$-$C_{10}$ qui peut être substitué par des groupes méthyle, hydroxy, méthoxy, acétoxy, le chlore, le brome ou des groupes nitro.

**4.** Procédé de préparation de composés de formule :

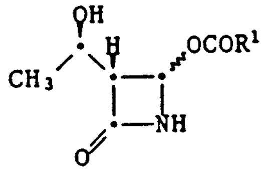

(I),

dans laquelle $R^1$ a les significations indiquées dans la revendication 1, caractérisé en ce que l'on soumet le composé de formule :

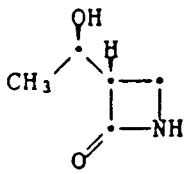

(V)

à acylation oxydative électrochimique en présence d'un acide $R^1COOH$ à des densités de courant de 10 à 400 mA/cm$^2$ et des températures de 0 à 50 ° C.

**5.** Procédé selon la revendication 4, pour préparer la (3R,4R,1'R)-4-acétoxy-3-(1'-hydroxyéthyl)-azétidinone de formule :

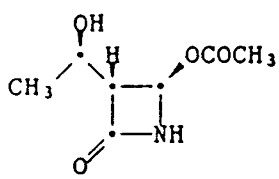

(Ia).

**6.** Procédé de préparation de composés de formule :

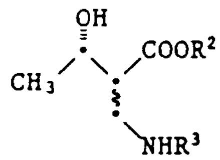

(IIIa),

dans laquelle $R^2$ et $R^3$ ont les significations indiquées dans la revendication 1, caractérisé en ce que l'on réduit un composé de formule :

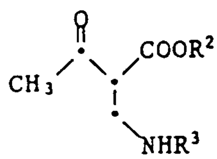

(II),

dans laquelle $R^2$ et $R^3$ ont les significations indiquées ci-dessus, ou un sel d'un tel composé, à l'aide de levure de boulanger et si on le désire,on convertit un composé obtenu répondant à la formule IIIa en un autre composé de formule IIIa selon l'invention et le cas échéant, on convertit un composé obtenu à l'état libre en un sel ou un sel obtenu en le composé libre ou en un autre sel.

**7.** Procédé selon la revendication 6, caractérisé en ce que le radical estérifiant $R^2$ et le radical acyle $R^3$ sont tels que définis dans la revendication 3.

**8.** Composés de formule :

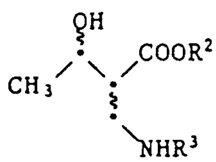

(III),

dans laquelle $R^2$ et $R^3$ ont les significations indiquées dans la revendication 1, leurs diastéréoisomères purs, les mélanges de deux diastéréoisomères, et leurs sels.

**9.** Composés selon la revendication 8, de formule III dans laquelle $R^2$ et $R^3$ représentent l'hydrogène ou ont les significations indiquées dans la revendication 3, leurs diastéréoisomères purs, les mélanges de deux diastéréoisomères, et leurs sels.

**10.** Composés selon la revendication 8, de formule :

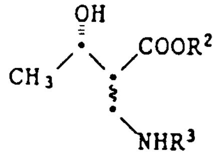

(IIIa),

dans laquelle $R^2$ représente l'hydrogène ou un groupe alkyle inférieur en $C_1$-$C_7$ et $R^3$ représente un groupe alcanoyle inférieur en $C_1$-$C_8$ ou (aryle en $C_1$-$C_{14}$)-carbonyle qui peut porter des substituants méthyle, méthoxy ou dinitro, leurs diastéréoisomères purs, et leurs sels.

**11.** Le composé selon la revendication 8, de formule :

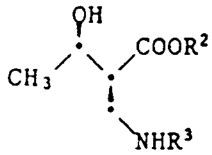

(IIIb),

dans laquelle $R^2$ et $R^3$ représentent l'hydrogène, et ses sels.

**12.** Composés de formule :

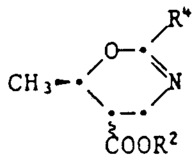

(IV),

dans laquelle $R^2$ et $R^4$ ont les significations indiquées dans la revendication 1, leurs diastéréoisomères purs, et leurs sels.

**13.** Composés selon la revendication 12, de formule IV dans laquelle $R^2$ représente un groupe alkyle inférieur en $C_1$-$C_4$ et $R^4$ un groupe alkyle inférieur en $C_1$-$C_4$ ou aryle en $C_6$-$C_{10}$, qui peut porter des substituants méthyle, hydroxy, méthoxy, acétoxy, chloro, bromo ou nitro, leurs diastéréoisomères purs, et leurs sels.

**14.** Le composé de formule :

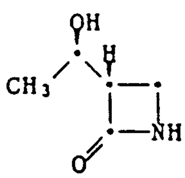

(V).

**Revendications pour l'Etat contractant suivant : ES**

**1.** Procédé de préparation des composés de formule :

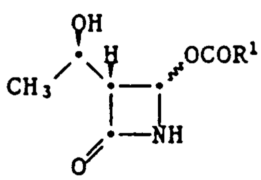

(I),

dans laquelle $R^1$ représente un groupe alkyle inférieur en $C_1$-$C_7$ ou aryle en $C_6$-$C_{14}$ éventuellement substitué par des groupes alkyle inférieurs en $C_1$-$C_7$, hydroxy, alcoxy inférieurs en $C_1$-$C_7$, alcanoyloxy

inférieurs en $C_1$-$C_8$, des halogènes ou des groupes nitro, caractérisé en ce que l'on réduit un composé de formule :

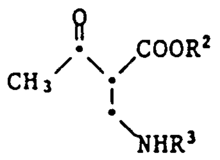

(II),

dans laquelle $R^2$ représente l'hydrogène ou un radical estérifiant et $R^3$ représente l'hydrogène ou un radical acyle choisi parmi les radicaux alcanoyle inférieurs en $C_1$-$C_8$, cycloalkylcarbonyle en $C_3$-$C_8$, arylcarbonyle en $C_6$-$C_{14}$, (alcoxy inférieur en $C_1$-$C_7$)-carbonyle, aryloxycarbonyle en $C_6$-$C_{14}$ et (aryle en $C_6$-$C_{14}$) - (alcoxy inférieur en $C_1$-$C_7$)-carbonyle, ou un sel d'un tel composé, à l'aide d'un agent réducteur énantiosélectif, ce qui donne un composé de formule :

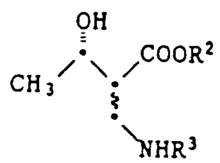

(IIIa),

qui, lorsque $R^2$ et/ou $R^3$ représentent l'hydrogène, est, dans un ordre quelconque, respectivement estérifié et acylé par un réactif introduisant le radical acyle $R^3$, et le composé de formule IIIa dans laquelle $R^2$ représente un radical estérifiant et $R^3$ un radical acyle $R^3$, ou un sel d'un tel composé, est cyclisé à l'aide d'un agent activant le groupe hydroxy, ce qui donne un composé de formule :

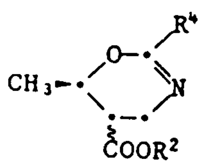

(IV),

dans laquelle $R^2$ représente un radical estérifiant et $R^4$ un groupe alkyle inférieur en $C_1$-$C_7$, cycloalkyle en $C_3$-$C_8$, aryle en $C_6$-$C_{14}$, alcoxy inférieur en $C_1$-$C_7$, aryloxy en $C_6$-$C_{14}$ ou (aryle en $C_6$-$C_{14}$) - alcoxy inférieur, les parties aryle pouvant être substituées comme indiqué en référence à $R^1$, on isomérise à l'aide d'une base, ce qui donne le diastéréoisomère de formule :

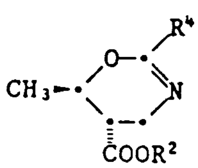

(IVa)

qu'on hydrolyse, ce qui donne le composé de formule :

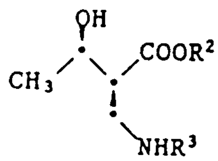

(IIIb),

dans laquelle $R^2$ et $R^3$ représentent l'hydrogène, ou un sel d'un tel composé, qu'on cyclise à l'aide d'un agent déshydratant, ce qui donne le composé de formule :

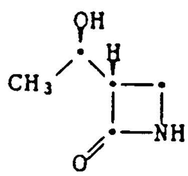

(V)

qu'on oxyde à l'aide d'un agent introduisant le radical $R^1COO$.

2. Procédé selon la revendication 1, pour la préparation de la (3R,4R,1'R)-4-acétoxy-3-(1'-hydroxyéthyl)-2azétidinone de formule :

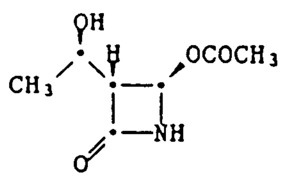

(Ia).

3. Procédé selon la revendication 1 ou 2, caractérisé en ce que le radical estérifiant $R^2$ est un radical alkyle inférieur en $C_1$-$C_4$, le radical acyle $R^3$ est un radical alcanoyle inférieur en $C_2$-$C_5$ ou arylcarbonyle en $C_6$-$C_{10}$ qui peut être substitué par des groupes méthyle, méthoxy ou dinitro, et $R^4$ représente respectivement un groupe alkyle inférieur en $C_1$-$C_4$ ou aryle en $C_6$-$C_{10}$ qui peut être substitué par des groupes méthyle, hydroxy, méthoxy, acétoxy, le chlore, le brome ou des groupes nitro.

4. Procédé de préparation de composés de formule :

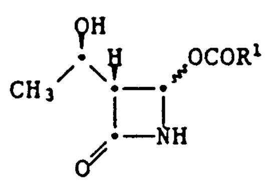

(I),

dans laquelle $R^1$ a les significations indiquées dans la revendication 1, caractérisé en ce que l'on soumet le composé de formule :

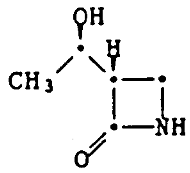 (V)

à acylation oxydative électrochimique en présence d'un acide R COOH à des densités de courant de 10 à 400 mA/cm$^2$ et des températures de 0 à 50 °C.

5. Procédé selon la revendication 4, pour préparer la (3R,4R,1'R)-4-acétoxy-3-(1'-hydroxyéthyl)-azétidinone de formule :

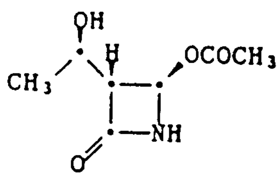 (Ia).

6. Procédé de préparation des composés de formule :

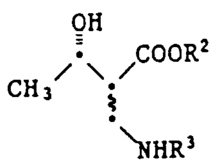 (IIIa),

dans laquelle R$^2$ et R$^3$ ont les significations indiquées dans la revendication 1, caractérisé en ce que l'on réduit un composé de formule :

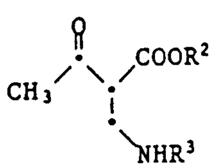 (II),

dans laquelle R$^2$ et R$^3$ ont les significations indiquées ci-dessus, ou un sel d'un tel composé à l'aide d'un agent réducteur énatiosélectif, et si on le désire, on convertit un composé obtenu répondant à la formule IIIa en un autre composé de formule IIIa selon l'invention, et le cas échéant, on convertit un composé obtenu à l'état libre en un sel ou un sel obtenu en un composé libre ou en un autre sel.

7. Procédé selon la revendication 6, caractérisé en ce que l'on utilise en tant qu'agent réducteur énantiosélectif la levure de boulanger.

8. Procédé selon la revendication 6 ou 7, caractérisé en ce que le radical estérifiant R$^2$ et le radical acyle R$^3$ sont tels que définis dans la revendication 3.

9. Procédé de préparation des composés de formule :

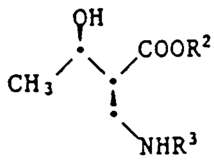

(IIIb),

dans laquelle $R^2$ et $R^3$ représentent l'hydrogène, et de leurs sels, caractérisé en ce que l'on hydrolyse un composé de formule :

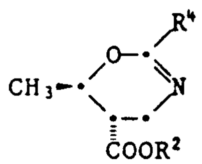

(IVa),

dans laquelle $R^2$ et R ont les significations indiquées dans la revendication 1, et si on le désire on convertit un composé obtenu à l'état libre en un sel ou un sel obtenu en un composé libre ou en un autre sel.

**10.** Procédé de préparation des composés de formule :

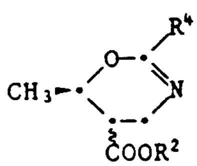

(IV),

dans laquelle $R^2$ et $R^4$ ont les significations indiquées dans la revendication 1, de leurs diastéréoisomères purs et de leurs sels, caractérisé en ce que l'on cyclise un composé de formule :

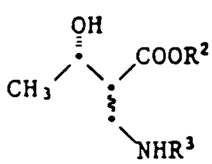

(IIIa),

dans laquelle $R^2$ et $R^3$ ont les significations indiquées dans la revendication 1, ou un sel d'un tel composé, à l'aide d'un agent activant les groupes hydroxy, et si on le désire, on isomérise un diastéréoisomère ou un mélange de diastéréoisomères ainsi obtenu à l'aide d'une base, et le cas échéant on convertit un composé obtenu à l'état libre en un sel ou un sel obtenu en un composé libre ou en un autre sel.

**11.** Procédé selon la revendication 10, caractérisé en ce que $R^2$ représente un groupe alkyle inférieur en $C_1$-$C_4$, $R^3$ représente un groupe alcanoyle inférieur en $C_1$-$C_4$ ou (aryle en $C_6$-$C_{10}$)-carbonyle, qui peut porter des substituants méthyle, méthoxy ou dinitro, et $R^4$ représente un groupe alkyle inférieur en $C_1$-$C_4$ ou aryle qui peut porter des substituants méthyle, hydroxy, méthoxy, acétoxy, chloro, bromo ou nitro.

**12.** Procédé de préparation du composé de formule :

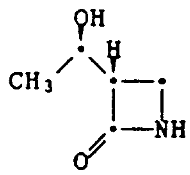

(V).

caractérisé en ce que l'on cyclise le composé de formule :

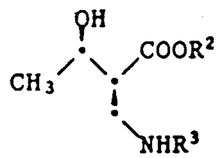

(IIIb),

dans laquelle $R^2$ et $R^3$ représentent l'hydrogène, ou un sel d'un tel composé, à l'aide d'un agent déshydratant.